# EUROPEAN PATENT SPECIFICATION

(11) **EP 2 172 548 B1**
(45) Date of publication and mention of the grant of the patent: **21.12.2016**
(21) Application number: 08778022.7
(22) Date of filing: 10.07.2008
(51) Int. Cl.: C12N 9/16, C12P 7/64

(54) **PHOSPHATIDIC ACID PHOSPHATASE HOMOLOGS AND USE THEREOF**
PHOSPHATIDSÄURE-PHOSPHATASE-HOMOLOGE UND IHRE VERWENDUNG
HOMOLOGUES DE L'ACIDE PHOSPHATIDIQUE PHOSPHATASE ET LEUR UTILISATION

(30) Priority: 11.07.2007 JP 2007181899
(43) Date of publication of application: 07.04.2010
(73) Proprietor: Suntory Holdings Limited, Kita-ku, Osaka-shi Osaka 530-8203 (JP)
(72) Inventor: OCHIAI, Misa, Mishima-gun Osaka 618-8503 (JP); TOKUDA, Hisanori, Mishima-gun Osaka 618-8503 (JP)
(74) Representative: Vossius & Partner Patentanwälte Rechtsanwälte mbB
(86) International application number: PCT/JP2008/062450
(87) International publication number: WO 2009/008466

(56) References cited:
- WO-A1-03/020936
- JP-A- 2005 287 403
- US-B1- 6 476 294
- GALAGAN J.E. ET AL.: 'The genome sequence of the filamentous fungus Neurospora crassa' NATURE vol. 422, no. 6934, 2003, pages 859 - 868, XP002365858
- PILLAI M.G. ET AL.: 'Characterization of triacylgylcerol biosynthesis in subcellular fractions of an oleaginous fungus, Mortierella ramanniana var. angulispora' BIOCHIM. BIOPHYS. ACTA vol. 1993, no. 1, 1998, pages 128 - 136, XP008131426
- OCHIAI M. ET AL.: 'Idenshi Kumikae ni yoru Shokubutsu Shibosan no Kaihen' NIPPON NOGEI KAGAKUKAI TAIKAI KOEN YOSHISHU 2002, page 273 + ABSTR. NO. 4-4DA20, XP008133552
- TAKENO S. ET AL.: 'Arachidonic acid Seisansei Shijokin Mortierella alpina 1S-4 ni Okeru Shibosan Sacho Enchoka Koso (GLELO) Idenshi Hatsugen ni yoru Shibosan Sosei no Kaihen' NIPPON NOGEI KAGAKUKAI TAIKAI KOEN YOSHISHU 2004, page 25 + ABSTR. NO. 2A07A10, XP008133551
- [Online] Retrieved from the Internet: <URL:http://www.chemicalbook.com/ChemicalPr oductProperty_EN_CB12141515.htm>

## Description

### TECHNICAL FIELD

This specification claims priority to Japanese Patent Application No. 2007-181899 (filed on July 11, 2007).

The present invention relates to novel genes for phosphatidic acid phosphatase.

### BACKGROUND ART

Fatty acids are important components of lipids such as phospholipids and triacylglycerols. Fatty acids containing two or more unsaturated bonds are collectively referred to as polyunsaturated fatty acids (PUFA) and are known to include arachidonic acid, dihomo-γ-linolenic acid, eicosapentaenoic acid and docosahexaenoic acid. Some of these polyunsaturated fatty acids cannot be synthesized in the animal body, and such polyunsaturated fatty acids should be taken as essential fatty acids from food sources. Polyunsaturated fatty acids are widely distributed. By way of example, arachidonic acid is separated from lipids extracted from the adrenal glands and/or livers of animals. However, the amount of polyunsaturated fatty acids contained in animal organs is small and cannot be obtained sufficiently for large supplies when simply extracted or separated from animal organs. For this reason, microbial techniques have been developed for obtaining polyunsaturated fatty acids by culturing various microorganisms. Above all, microorganisms of the genus *Mortierella* are known to produce lipids containing arachidonic acid and other polyunsaturated fatty acids.

Other attempts have also been made to produce polyunsaturated fatty acids in plants. Polyunsaturated fatty acids constitute storage lipids such as triacylglycerols and are known to be accumulated within microorganism cells or plant seeds.

Triacylglycerols, which are storage lipids, are produced in vivo as follows. Acyl group transfer occurs on glycerol-3-phosphate by glycerol-3-phosphate acyltransferase to form lysophosphatidic acid, on which acyl group transfer further occurs by lysophosphatidic acid acyltransferase to form phosphatidic acid. This phosphatidic acid is, in turn, dephosphorylated by phosphatidic acid phosphatase to form diacylglycerol, on which acyl group transfer then occurs by diacylglycerol acyltransferase to form triacylglycerol.

In the above pathway, phosphatidic acid (hereinafter also referred to as "PA" or "1,2-diacyl-sn-glycerol-3-phosphate") serves not only as a precursor of triacylglycerol, but also as a precursor of diacyl-type glycerophospholipid biosynthesis. In yeast or other microbes, phosphatidic acid cytidyltransferase acts on PA and cytidine 5'-triphosphate (CTP) to synthesize CDP-diacylglycerol (CDP-DG), which in turn is used for biosynthesis of various phospholipids.

As described above, dephosphorylation of PA for biosynthesis of diacylglycerol (hereinafter also referred to as "DG") is known to be catalyzed by phosphatidic acid phosphatase (E.C. 3.1.3.4; hereinafter also referred to as "PAP"). This PAP enzyme is known to be present in all organisms ranging from bacteria to vertebrate animals. Moreover, in fungi, PAP is a key enzyme for biosynthesis of triacylglycerols (which are storage lipids) starting from PA.

The yeast *Saccharomyces cerevisiae,* which is a fungus, is known to have two types of PAP (Non-patent Document 1). One is Mg²⁺-dependent PAP (PAP1) and the other is Mg²⁺-independent PAP (PAP2). As a gene encoding the former, the PAH1 gene has been known. However, since pah1Δ mutants have PAP1 activity, there are other potential genes responsible for PAP1 activity. On the other hand, as genes encoding the latter, the DPP1 and LPP1 genes have been known and they are substantially responsible for PAP2 activity in yeast. Enzymes encoded by these genes have wide substrate specificity and are known to also act on diacylglycerol pyrophosphate (DGPP), lysophosphatidic acid, sphingoid base phosphate, isoprenoid phosphate and so on to cause dephosphorylation thereof.

In other fungi, the presence of these homologs has been confirmed, but there is no report of their functions, etc.

In a lipid-producing fungus, *Mortierella alpina,* its microsomal fraction has been known to have PAP activity (Non-patent Document 2).
Non-patent Document 1: Trends Biochem. Sci., 31(12), 694-699, 2006
Non-patent Document 2: Biochemical Society Transactions, 28, 707-709, 2000

### DISCLOSURE OF THE INVENTION

### PROBLEMS TO BE SOLVED BY THE INVENTION

However, PAP genes previously reported have not been studied for their ability to alter the fatty acid rate of fatty acid compositions produced by host cells in which these PAP genes are introduced and expressed. There is a need to identify a novel gene which allows production of fats and oils with a desired fatty acid rate and/or enrichment of desired fatty acids by being introduced into or expressed in host cells.

### MEANS FOR SOLVING THE PROBLEMS

The object of the present invention is to provide a protein or nucleic acid which allows production of fats and oils with a desired fatty acid rate and/or enrichment of desired fatty acids by being expressed in or introduced into host cells.

To achieve the above object, the inventors of the present invention have made extensive and intensive efforts. First, EST analysis was performed on a lipid-producing fungus, *Mortierella alpina,* to extract sequences sharing high identity with known PAP genes. To obtain the entire open reading frame (ORF) encoding PAP, genes were further cloned by cDNA library screening or PCR. As a result of attempting to introduce these genes into highly proliferative host cells (e.g., yeast cells) to thereby produce a desired fatty acid composition, the inventors succeeded in cloning a gene related to a novel PAP which allows production of a fatty acid composition different from that produced by a host not carrying the gene. This led to the completion of the present invention. Namely, the present invention is as follows.
(1) A protein shown in any one of (a) to (e) below:
   (a) a protein consisting of the amino acid sequence shown in SEQ ID NO: 2;
   (b) a protein which consists of an amino acid sequence with deletion, substitution or addition of 1 to 50 amino acids in SEQ ID NO: 2 and which has phosphatidic acid phosphatase activity;
   (c) a protein which consists of an amino acid sequence sharing an identity of 85% or more with the amino acid sequence consisting of SEQ ID NO: 2 and which has phosphatidic acid phosphatase activity;
   (d) a protein which consists of an amino acid sequence with deletion, substitution or addition of 1 to 50 amino acids in SEQ ID NO: 2 and which has the ability to yield a fatty acid rate ensuring a higher ratio of at least one or more of (i) to (vi) shown below in the fatty acid rate of a host expressing a protein consisting of the amino acid sequence than in the fatty acid rate of a host not expressing the protein:
      (i) the oleic acid content;
      (ii) the ratio of the palmitoleic acid content to the palmitic acid content;
      (iii) the ratio of the oleic acid content to the palmitic acid content;
      (iv) the ratio of the total content of stearic acid and oleic acid to the palmitic acid content;
      (v) the ratio of the C₁₈ fatty acid content to the C₁₆ fatty acid content; and
      (vi) the ratio of the arachidonic acid, dihomo-γ-linolenic acid and/or γ-linolenic acid content; or
   (e) a protein which consists of an amino acid sequence sharing an identity of 85% or more with the amino acid sequence consisting of SEQ ID NO: 2 and which has the ability to yield a fatty acid rate ensuring a higher ratio of at least one or more of (i) to (vi) shown below in the fatty acid rate of a host expressing a protein consisting of the amino acid sequence than in the fatty acid rate of a host not expressing the protein:
      (i) the oleic acid content;
      (ii) the ratio of the palmitoleic acid content to the palmitic acid content;
      (iii) the ratio of the oleic acid content to the palmitic acid content;
      (iv) the ratio of the total content of stearic acid and oleic acid to the palmitic acid content;
      (v) the ratio of the C₁₈ fatty acid content to the C₁₆ fatty acid content; and
      (vi) the ratio of the arachidonic acid, dihomo-γ-linolenic acid and/or γ-linolenic acid content.
(2) A nucleic acid comprising a nucleotide sequence shown in any one of (a) to (i) below:
   (a) the nucleotide sequence shown in SEQ ID NO: 4;
   (b) the nucleotide sequence shown in SEQ ID NO: 1;
   (c) a nucleotide sequence which encodes a protein according to (1)(a) above;
   (d) a nucleotide sequence which encodes a protein according to (1)(b) above;
   (e) a nucleotide sequence which encodes a protein according to (1)(c) above;
   (f) a nucleotide sequence which encodes a protein according to (1)(d) above;
   (g) a nucleotide sequence which encodes a protein according to (1)(e) above;
   (h) a nucleotide sequence which consists of a nucleotide sequence sharing an identity of 85% or more with the nucleotide sequence consisting of SEQ ID NO: 4 and which encodes a protein having phosphatidic acid phosphatase activity; or
   (i) a nucleotide sequence which consists of a nucleotide sequence sharing an identity of 85% or more with the nucleotide sequence consisting of SEQ ID NO: 4 and which encodes the following protein:
      a protein which has the ability to yield a fatty acid rate ensuring a higher ratio of at least one or more of (i) to (vi) shown below in the fatty acid rate of a host expressing the protein than in the fatty acid rate of a host not expressing the protein:
         (i) the oleic acid content;
         (ii) the ratio of the palmitoleic acid content to the palmitic acid content;
         (iii) the ratio of the oleic acid content to the palmitic acid content;
         (iv) the ratio of the total content of stearic acid and oleic acid to the palmitic acid content;
         (v) the ratio of the C₁₈ fatty acid content to the C₁₆ fatty acid content; and
         (vi) the ratio of the arachidonic acid, dihomo-γ-linolenic acid and/or γ-linolenic acid content.
(3) A nucleic acid according to (2) above comprising a nucleotide sequence shown in any one of (a) to (d) below:
   (a) the nucleotide sequence according to (2)(d) above, wherein the number of amino acids to be deleted, substituted or added in the protein according to (1)(b) above is 1 to 10;
   (b) the nucleotide sequence according to (2)(e) above, wherein the amino acid sequence of the protein according to (1)(c) above shares an identity of 90% or more with the amino acid sequence consisting of SEQ ID NO: 2;
   (c) the nucleotide sequence according to (2)(f) above, wherein the number of amino acids to be deleted, substituted or added in the protein according to (1)(d) above is 1 to 10; or
   (d) the nucleotide sequence according to (2)(g) above, wherein the amino acid sequence of the protein according to (1)(e) above shares an identity of 90% or more with the amino acid sequence consisting of SEQ ID NO: 2.
(4) A recombinant vector comprising the nucleic acid according to (2) above or (3) above.
(5) A transformant transformed with the recombinant vector according to (4) above.
6. A method for preparing a fatty acid composition comprising culturing the transformant according to (5) above and collecting the fatty acid composition contained in the cultured product obtained by culturing the transformant according to (5) above.

In addition, the present disclosure relates to:
(1) A nucleic acid comprising a nucleotide sequence shown in any one of (a) to (e) below:
   (a) a nucleotide sequence which encodes a protein consisting of an amino acid sequence with deletion, substitution or addition of one or more amino acids in the amino acid sequence shown in SEQ ID NO: 2 and having phosphatidic acid phosphatase activity;
   (b) a nucleotide sequence which is hybridizable under stringent conditions with a nucleic acid consisting of a nucleotide sequence complementary to a nucleotide sequence consisting of SEQ ID NO: 4 and which encodes a protein having phosphatidic acid phosphatase activity;
   (c) a nucleotide sequence which consists of a nucleotide sequence sharing an identity of 70% or more with a nucleotide sequence consisting of SEQ ID NO: 4 and which encodes a protein having phosphatidic acid phosphatase activity;
   (d) a nucleotide sequence which encodes an amino acid sequence sharing an identity of 70% or more with an amino acid sequence consisting of SEQ ID NO: 2 and which encodes a protein having phosphatidic acid phosphatase activity; or
   (e) a nucleotide sequence which is hybridizable under stringent conditions with a nucleic acid consisting of a nucleotide sequence complementary to a nucleotide sequence encoding a protein consisting of the amino acid sequence shown in SEQ ID NO: 2 and which encodes a protein having phosphatidic acid phosphatase activity.
(2) The nucleic acid according to (1) above, which comprises a nucleotide sequence shown in any one of (a) to (c) below:
   (a) a nucleotide sequence which encodes a protein consisting of an amino acid sequence with deletion, substitution or addition of 1 to 10 amino acids in the amino acid sequence shown in SEQ ID NO: 2 and having phosphatidic acid phosphatase activity;
   (b) a nucleotide sequence which is hybridizable under conditions of 2 x SSC at 50°C with a nucleic acid consisting of a nucleotide sequence complementary to a nucleotide sequence consisting of SEQ ID NO: 4 and which encodes a protein having phosphatidic acid phosphatase activity; or
   (c) a nucleotide sequence which encodes an amino acid sequence sharing an identity of 90% or more with an amino acid sequence consisting of SEQ ID NO: 2 and which encodes a protein having phosphatidic acid phosphatase activity.
(3) A nucleic acid comprising a nucleotide sequence shown in any one of (a) to (c) below or a fragment thereof:
   (a) the nucleotide sequence shown in SEQ ID NO: 4;
   (b) a nucleotide sequence encoding a protein consisting of the amino acid sequence shown in SEQ ID NO: 2; or
   (c) the nucleotide sequence shown in SEQ ID NO: 1.
(4) A nucleic acid comprising a nucleotide sequence shown in any one of (a) to (e) below:
   (a) a nucleotide sequence which encodes the following protein:
      a protein which consists of an amino acid sequence with deletion, substitution or addition of one or more amino acids in the amino acid sequence shown in SEQ ID NO: 2 and which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content;
   (b) a nucleotide sequence which is hybridizable under stringent conditions with a nucleic acid consisting of a nucleotide sequence complementary to a nucleotide sequence consisting of SEQ ID NO: 4 and which encodes the following protein:
      a protein which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content;
   (c) a nucleotide sequence which consists of a nucleotide sequence sharing an identity of 70% or more with a nucleotide sequence consisting of SEQ ID NO: 4 and which encodes the following protein:
      a protein which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content;
   (d) a nucleotide sequence which encodes the following protein:
      a protein which consists of an amino acid sequence sharing an identity of 70% or more with an amino acid sequence consisting of SEQ ID NO: 2 and which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content; or
   (e) a nucleotide sequence which is hybridizable under stringent conditions with a nucleic acid consisting of a nucleotide sequence complementary to a nucleotide sequence encoding a protein consisting of the amino acid sequence shown in SEQ ID NO: 2 and which encodes the following protein:
      a protein which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content.
(5) The nucleic acid according to (4) above, which comprises a nucleotide sequence shown in any one of (a) to (c) below:
   (a) a nucleotide sequence which encodes the following protein:
      a protein which consists of an amino acid sequence with deletion, substitution or addition of 1 to 10 amino acids in the amino acid sequence shown in SEQ ID NO: 2 and which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content;
   (b) a nucleotide sequence which is hybridizable under conditions of 2 x SSC at 50°C with a nucleic acid consisting of a nucleotide sequence complementary to a nucleotide sequence consisting of SEQ ID NO: 4 and which encodes the following protein:
      a protein which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content; or
   (c) a nucleotide sequence which encodes the following protein:
      a protein which consists of an amino acid sequence sharing an identity of 90% or more with an amino acid sequence consisting of SEQ ID NO: 2 and which has the ability to yield a fatty acid rate ensuring a higher ratio of at least one or more of i) to vi) shown below in the fatty acid rate of a host expressing the protein than in the fatty acid rate of a host not expressing the protein:
         i) the oleic acid content;
         ii) the ratio of the palmitoleic acid content to the palmitic acid content;
         iii) the ratio of the oleic acid content to the palmitic acid content;
         iv) the ratio of the total content of stearic acid and oleic acid to the palmitic acid content;
         v) the ratio of the C₁₈ fatty acid content to the C₁₆ fatty acid content; and
         vi) the ratio of the arachidonic acid, dihomo-γ-linolenic acid and/or γ-linolenic acid content.
(6) A protein shown in (a) or (b) below:
   (a) a protein which consists of an amino acid sequence with deletion, substitution or addition of one or more amino acids in SEQ ID NO: 2 and which has phosphatidic acid phosphatase activity; or
   (b) a protein which consists of an amino acid sequence sharing an identity of 70% or more with an amino acid sequence consisting of SEQ ID NO: 2 and which has phosphatidic acid phosphatase activity.
(7) A protein shown in (a) or (b) below:
   (a) a protein which consists of an amino acid sequence with deletion, substitution or addition of one or more amino acids in SEQ ID NO: 2 and which has the ability to yield a fatty acid rate ensuring a higher ratio of at least one or more of i) to vi) shown below in the fatty acid rate of a host expressing a protein consisting of the amino acid sequence than in the fatty acid rate of a host not expressing the protein:
      i) the oleic acid content;
      ii) the ratio of the palmitoleic acid content to the palmitic acid content;
      iii) the ratio of the oleic acid content to the palmitic acid content;
      iv) the ratio of the total content of stearic acid and oleic acid to the palmitic acid content;
      v) the ratio of the C₁₈ fatty acid content to the C₁₆ fatty acid content; and
      vi) the ratio of the arachidonic acid, dihomo-γ-linolenic acid and/or γ-linolenic acid content; or
   (b) a protein which consists of an amino acid sequence sharing an identity of 70% or more with an amino acid sequence consisting of SEQ ID NO: 2 and which has the ability to yield a fatty acid rate ensuring a higher ratio of at least one or more of i) to vi) shown below in the fatty acid rate of a host expressing a protein consisting of the amino acid sequence than in the fatty acid rate of a host not expressing the protein:
      i) the oleic acid content;
      ii) the ratio of the palmitoleic acid content to the palmitic acid content;
      iii) the ratio of the oleic acid content to the palmitic acid content;
      iv) the ratio of the total content of stearic acid and oleic acid to the palmitic acid content;
      v) the ratio of the C₁₈ fatty acid content to the C₁₆ fatty acid content; and
      vi) the ratio of the arachidonic acid, dihomo-γ-linolenic acid and/or γ-linolenic acid content.
(8) A protein consisting of the amino acid sequence shown in SEQ ID NO: 2.
(9) A recombinant vector comprising the nucleic acid according to any one of (1) to (5) above.
(10) A transformant transformed with the recombinant vector according to (9) above.
(11) A fatty acid composition obtained by culturing the transformant according to (10) above, wherein at least one or more of i) to vi) shown below is higher in the fatty acid rate of the fatty acid composition than in a cultured product obtained by culturing a host which is not transformed with the recombinant vector according to (9) above:
   i) the oleic acid content;
   ii) the ratio of the palmitoleic acid content to the palmitic acid content;
   iii) the ratio of the oleic acid content to the palmitic acid content;
   iv) the ratio of the total content of stearic acid and oleic acid to the palmitic acid content;
   v) the ratio of the C₁₈ fatty acid content to the C₁₆ fatty acid content; and
   vi) the ratio of the arachidonic acid, dihomo-γ-linolenic acid and/or γ-linolenic acid content.
(12) A method for preparing a fatty acid composition, which comprises collecting the fatty acid composition according to (11) above from a cultured product obtained by culturing the transformant according to (10) above.
(13) A food product comprising the fatty acid composition according to (11) above. ADVANTAGES OF THE INVENTION

The PAP of the present invention allows a host to produce a fatty acid composition whose fatty acid rate differs from that of a fatty acid composition produced by a host not carrying PAP. As a result, the PAP of the present invention enables the provision of lipids having desired properties and effects, and is useful as being applicable to foods, cosmetics, pharmaceuticals, soaps, etc.

Moreover, the PAP of the present invention allows improvement in the ability to produce fatty acids and storage lipids, and hence is preferred as a means for improving the productivity of polyunsaturated fatty acids in microorganisms and plants.

### BRIEF DESCRIPTION OF THE DRAWINGS

Figure 1 shows the cDNA sequence of MaPAP1 according to the present invention, along with its deduced amino acid sequence.
Figure 2 shows an alignment of the deduced amino acid sequence of MaPAP1p (SEQ ID NO: 2) with the amino acid sequences of PAP2 family proteins. In the figure, the three double-underlined segments represent consensus regions conserved among PAP2 family enzymes, and "*" represents an amino acid residue essential for PAP activity. BEST MODE FOR CARRYING OUT THE INVENTION

The present invention relates to novel genes for phosphatidic acid phosphatase derived from the genus *Mortierella,* characterized by dephosphorylating phosphatidic acid to generate diacylglycerol.

Phosphatidic acid phosphatase (PAP) in the present invention is an enzyme that catalyzes a reaction in which phosphatidic acid is dephosphorylated to generate diacylglycerol. A substrate for PAP in the present invention is generally, but not limited to, phosphatidic acid.

### Nucleic acids of the present invention encoding phosphatidic acid phosphatase

Phosphatidic acid phosphatase (PAP) in the present invention encompasses MaPAP1. The correspondence between cDNA, CDS, ORF and amino acid sequences of a nucleic acid encoding MaPAP1 is summarized in Table 1 below.

**[Table 1]**

| | MaPAP1 | |
|---|---|---|
| | SEQ ID NO: | Corresponding region in SEQ ID NO: 1 |
| cDNA | SEQ ID NO: 1 | ***** |
| CDS | SEQ ID NO: 3 | Positions 105-1223 |
| ORF | SEQ ID NO: 4 | Positions 105-1220 |
| Amino acid sequence | SEQ ID NO: 2 | ***** |

Namely, sequences related to the PAP of the present invention include SEQ ID NO: 2 (amino acid sequence of MaPAP1), SEQ ID NO: 4 (sequence representing the ORF region of MaPAP1), SEQ ID NO: 3 (sequence representing the CDS region of MaPAP1) and SEQ ID NO: 1 (nucleotide sequence of cDNA for MaPAP1). Among them, SEQ ID NO: 3 corresponds to nucleotides 105-1223 of SEQ ID NO: 1, while SEQ ID NO: 4 corresponds to nucleotides 105-1220 of SEQ ID NO: 1 or nucleotides 1-1116 of SEQ ID NO: 3.

The nucleic acids of the present invention encompass single-stranded and double-stranded DNAs as well as complementary RNAs thereof, which may be either naturally occurring or artificially prepared. DNAs include, but are not limited to, genomic DNAs, cDNAs corresponding to the genomic DNAs, chemically synthesized DNAs, PCR-amplified DNAs, as well as combinations thereof and DNA/RNA hybrids.

Preferred embodiments for the nucleic acids of the present invention include (a) the nucleotide sequence shown in SEQ ID NO: 4, (b) a nucleotide sequence encoding a protein consisting of the amino acid sequence shown in SEQ ID NO: 2, and (c) the nucleotide sequence shown in SEQ ID NO: 1.

The above nucleotide sequence shown in SEQ ID NO: 4, nucleotide sequence encoding a protein consisting of the amino acid sequence shown in SEQ ID NO: 2, and nucleotide sequence shown in SEQ ID NO: 1 are as shown in Table 1.

To obtain these nucleotide sequences, nucleotide sequence data of ESTs or genomic DNAs from organisms having PAP activity may be used to search a nucleotide sequence encoding a protein sharing high identity with known proteins having PAP activity. Preferred organisms having PAP activity are lipid-producing fungi including, but not limited to, *M. alpina.*

For EST analysis, a cDNA library is first prepared. As to techniques for cDNA library preparation, reference may be made to "Molecular Cloning, A Laboratory Manual 3rd ed." (Cold Spring Harbor Press (2001)). Alternatively, a commercially available cDNA library preparation kit may be used. Techniques for cDNA library preparation suitable for the present invention are as follows, by way of example. Namely, an appropriate strain of *M. alpina,* a lipid-producing fungus, is inoculated into an appropriate medium and pre-cultured for an appropriate period. Culture conditions suitable for this pre-culture include, for example, medium composition of 1.8% glucose, 1% yeast extract and pH 6.0, a culture period of 3 days, and a culture temperature of 28°C. The pre-cultured product is then subjected to main culture under appropriate conditions. Medium composition suitable for main culture may be, for example, 1.8% glucose, 1% soybean powder, 0.1 % olive oil, 0.01% Adekanol, 0.3% KH₂PO₄, 0.1% Na₂SO₄, 0.05% CaCl₂·2H₂O, 0.05% MgCl₂·6H₂O and pH 6.0. Culture conditions suitable for main culture may be, for example, aerobic spinner culture at 300 rpm, 1 vvm, 26°C for 8 days. An appropriate amount of glucose may be added during culture. The cultured product is sampled at appropriate time points during main culture, from which the cells are then collected to prepare total RNA. For preparation of total RNA, it is possible to use any known technique, such as guanidine hydrochloride/CsCl method. The resulting total RNA may be treated with a commercially available kit to purify poly(A)⁺RNA. Further, a cDNA library may be prepared with a commercially available kit. Then, any clone from the cDNA library thus prepared is determined for its nucleotide sequence by using primers which are designed on a vector to allow determination of the nucleotide sequence of an insert. As a result, ESTs can be obtained. For example, when a ZAP-cDNA GigapackIII Gold Cloning Kit (STRATAGENE) is used for cDNA library preparation, directional cloning can be performed.

When analyzed by BLASTX, the MaPAP1 gene of the present invention shares 57.4% identity with a nucleotide sequence encoding a *Neurospora crassa*-derived diacylglycerol pyrophosphate phosphatase (DPP1) homolog (Accession No. CAD70721) having the lowest E-value (i.e., showing the highest identity) and shares 59.3% identity with the amino acid sequence of this homolog.

The present invention also encompasses nucleic acids functionally equivalent to a nucleic acid comprising the above nucleotide sequence shown in SEQ ID NO: 4 (hereinafter also referred to as "the nucleotide sequence of the present invention") or nucleotide sequence encoding a protein consisting of the amino acid sequence shown in SEQ ID NO: 2 (hereinafter also referred to as "the amino acid sequence of the present invention"). The phrase "functionally equivalent" is intended to mean that a protein encoded by the nucleotide sequence of the present invention or a protein consisting of the amino acid sequence of the present invention has PAP activity. In addition to this PAP activity, a protein encoded by the nucleotide sequence of the present invention or a protein consisting of the amino acid sequence of the present invention may have the ability to yield a fatty acid rate ensuring a higher ratio of at least one or more of:
i) the oleic acid content;
ii) the ratio of the palmitoleic acid content to the palmitic acid content;
iii) the ratio of the oleic acid content to the palmitic acid content;
iv) the ratio of the total content of stearic acid and oleic acid to the palmitic acid content;
v) the ratio of the C₁₈ fatty acid content to the C₁₆ fatty acid content; and
vi) the ratio of the arachidonic acid, dihomo-γ-linolenic acid and/or γ-linolenic acid content
in the fatty acid rate of a host expressing the protein than in the fatty acid rate of a host not expressing the protein (such a protein is hereinafter also referred to as a "protein having the ability to yield the fatty acid rate of PAP in the present invention").

A specific example is a nucleic acid comprising a nucleotide sequence encoding a protein having the ability to yield a fatty acid rate satisfying at least one or more of the following:
i) the oleic acid content is 46% or more, preferably 48% or more, 50% or more, 52% or more, 54% or more;
ii) the ratio of the palmitoleic acid content to the palmitic acid content is 7.0 or more, preferably 8.0 or more, 8.5 or more;
iii) the ratio of the oleic acid content to the palmitic acid content is 7.5 or more, preferably 8.0 or more, 10.0 or more, 12.0 or more, 13.0 or more;
iv) the ratio of the total content of stearic acid and oleic acid to the palmitic acid content is 8.0 or more, preferably 10.0 or more, 12.0 or more, 14.0 or more, 14.5 or more;
v) the ratio of the C₁₈ fatty acid content to the C₁₆ fatty acid content is 1.1 or more, preferably 1.2 or more, 1.3 or more, 1.4 or more; and
vi) the arachidonic acid content is 0.45 or more, preferably 0.50 or more, 0.53 or more, 0.55 or more, the dihomo-γ-linolenic acid content is 0.34 or more, preferably 0.40 or more, 0.42 or more, 0.45 or more, and/or the γ-linolenic acid content is 0.55 or more, preferably 0.65 or more, 0.67 or more, 0.70 or more, 0.75 or more (the above numerics each denote a ratio to total fatty acid when the PAP gene of the present invention is expressed in arachidonic acid-producing yeast cells),
when the above nucleotide sequence of the present invention is inserted into expression vector pYE22m (Biosci. Biotech. Biochem., 59, 1221-1228, 1995) and transformed into a yeast host, *Saccharomyces cerevisiae* strain EH13-15 (Appl. Microbiol. Biotechnol., 30, 515-520, 1989), and the resulting transformant is cultured to collect the cells, which are then analyzed for fatty acids by the procedures described in Example 6 below. More preferred is a nucleic acid comprising a nucleotide sequence encoding a protein having both PAP activity and the above ability to yield the fatty acid rate of PAP in the present invention.

One of the characteristic features in the fatty acid composition disclosed herein is high arachidonic acid content. Arachidonic acid, a substance represented by the chemical formula C₂₀H₃₂O₂ and having a molecular weight of 304.47, is a carboxylic acid containing 20 carbon atoms and 4 double bonds ([20:4(n-6)]) and classified as a member of the (n-6) series. Arachidonic acid is present as an important phospholipid (particularly phosphatidylethanolamine, phosphatidylcholine, phosphatidylinositol) in animal cell membranes and is contained in abundance in the brain. Moreover, arachidonic acid serves as a starting material for a series of eicosanoids (e.g., prostaglandin, thromboxane, leukotriene) generated by the arachidonic acid cascade, and is also important as a second messenger in intercellular signaling. On the other hand, arachidonic acid is synthesized from linolic acid in the animal body. However, depending on their species or age, some animals do not exert this function sufficiently to produce the required amount of arachidonic acid or have no function to produce arachidonic acid. Thus, arachidonic acid should be taken from food sources and can be regarded as an essential fatty acid.

The arachidonic acid content in the fatty acid composition disclosed herein may be measured as follows, by way of example. Namely, a plasmid for PAP of the present invention is inserted into a vector such as pDuraSC or pDura5MCS, as described in Example 8, and transformed into a *M*. *alpina* strain. The resulting transformant is allowed to express and cultured according to the procedures described in Example 8. The cultured cells thus obtained are used to measure the fatty acid content in the cells and/or the arachidonic acid content per medium, etc. To analyze the arachidonic acid content, etc., for example, fatty acids in the resulting cultured cells are derived into corresponding fatty acid methyl esters by the hydrochloric acid/methanol method, and then extracted with hexane. After distilling off hexane, the fatty acids are analyzed by gas chromatography. According to this analysis, *M. alpina* transformed with the PAP of the present invention has been found to show not only high fatty acid content in the cells, but also high arachidonic acid production per medium. Thus, the fatty acid composition disclosed herein having high arachidonic acid content is preferred because it enables the efficient intake of arachidonic acid.

Another characteristic feature in the fatty acid composition disclosed herein is high dihomo-γ-linolenic acid content. Dihomo-γ-linolenic acid (DGLA), a substance represented by the chemical formula C₂₀H₃₄O₂ and having a molecular weight of 306.48, is a carboxylic acid containing 20 carbon atoms and 3 double bonds ([20:3(n-6)]) and classified as a member of the (n-6) series. DGLA is obtained by elongation of γ-linolenic acid (18:3(n-6)). Upon addition of one more double bond to DGLA, arachidonic acid is generated.

Yet another characteristic feature in the fatty acid composition disclosed herein is high γ-linolenic acid content. γ-Linolenic acid, a substance represented by the chemical formula C₁₈H₃₀O₂ and having a molecular weight of 278.436, is a carboxylic acid containing 18 carbon atoms and 3 double bonds ([18:3(n-6)]) and classified as a member of the (n-6) series. Humans have the ability to produce γ-linolenic acid from linolic acid ([18:2(n-6)]) through Δ6 desaturase-catalyzed dehydrogenation, but they often take it from foods.

Such nucleic acids that are functionally equivalent to the nucleic acids of the present invention include a nucleic acid comprising a nucleotide sequence shown in any one of (a) to (e) below. It should be noted that when used to describe the nucleotide sequences listed below, the phrase "the above activity of the present invention" is intended to mean "PAP activity and/or the ability to yield the fatty acid rate of PAP in the present invention" defined above.

(a) A nucleotide sequence which encodes a protein consisting of an amino acid sequence with deletion, substitution or addition of one or more amino acids in the amino acid sequence shown in SEQ ID NO: 2 and having the above activity of the present invention.

Nucleotide sequences contained in the nucleic acids of the present invention include a nucleotide sequence which encodes a protein consisting of an amino acid sequence with deletion, substitution or addition of one or more amino acids in the amino acid sequence shown in SEQ ID NO: 2 and having the above activity of the present invention.

More specifically, it is a nucleotide sequence which encodes a protein consisting of:
(i) an amino acid sequence with deletion of 1-50, amino acids in the amino acid sequence shown in SEQ ID NO: 2;
(ii) an amino acid sequence with substitution of other amino acids for 1-50, amino acids in the amino acid sequence shown in SEQ ID NO: 2;
(iii) an amino acid sequence with addition of other 1-50, amino acids in the amino acid sequence shown in SEQ ID NO: 2; or
(iv) an amino acid sequence with any combination of (i) to (iii) above, and having the above activity of the present invention.

Among the above modifications, substitution is preferably conservative, which means the replacement of a certain amino acid residue by another residue having similar physical and chemical characteristics. It may be any substitution as long as it does not substantially alter the structural characteristics of the original sequence. For example, any substitution is possible as long as the substituted amino acids do not disrupt a helix present in the original sequence or do not disrupt any other type of secondary structure characterizing the original sequence.

Conservative substitution is generally introduced by synthesis in biological systems or chemical peptide synthesis, preferably by chemical peptide synthesis. In this case, substituents may include unnatural amino acid residues, as well as peptidomimetics, and reversed or inverted forms of amino acid sequences in which unsubstituted regions are reversed or inverted.

Amino acid residues are classified and listed below in groups of mutually substitutable members, but are not limited to the following:
Group A: leucine, isoleucine, norleucine, valine, norvaline, alanine, 2-aminobutanoic acid, methionine, O-methylserine, t-butylglycine, t-butylalanine and cyclohexylalanine;
Group B: aspartic acid, glutamic acid, isoaspartic acid, isoglutamic acid, 2-aminoadipic acid and 2-aminosuberic acid;
Group C: asparagine and glutamine;
Group D: lysine, arginine, ornithine, 2,4-diaminobutanoic acid and 2,3-diaminopropionic acid;
Group E: proline, 3-hydroxyproline and 4-hydroxyproline;
Group F: serine, threonine and homoserine; and
Group G: phenylalanine and tyrosine.

Non-conservative substitution may involve the exchange of a member of one of the above classes for a member from another class. In this case, for the purpose of maintaining biological functions of the proteins of the present invention, it is preferable to consider the hydropathic index of amino acids (hydropathic amino acid index) (Kyte et al., J. Mol. Biol., 157:105-131(1982)).

In the case of non-conservative substitution, amino acid substitutions may also be accomplished on the basis of hydrophilicity.

In the specification and drawings of the present application, nucleotides, amino acids and abbreviations thereof are those according to the IUPAC-IUB Commission on Biochemical Nomenclature or those conventionally used in the art, for example, as described in Immunology--A Synthesis (second edition, edited by E.S. Golub and D.R. Gren, Sinauer Associates, Sunderland, Massachusetts (1991)). Moreover, amino acids which may have optical isomers are intended to represent their L-isomer, unless otherwise specified.

Stereoisomers (e.g., D-amino acids) of the above amino acids, unnatural amino acids such as α,α-disubstituted amino acids, N-alkylamino acids, lactic acid, and other unconventional amino acids may also be members constituting the proteins of the present invention.

It should be noted that in the protein notation used herein, the lefthand direction is the amino terminal direction and the righthand direction is the carboxy terminal direction, in accordance with standard usage and convention.

Similarly, unless otherwise specified, the lefthand end of single-stranded polynucleotide sequences is the 5'-end and the lefthand direction of double-stranded polynucleotide sequences is referred to as the 5' direction.

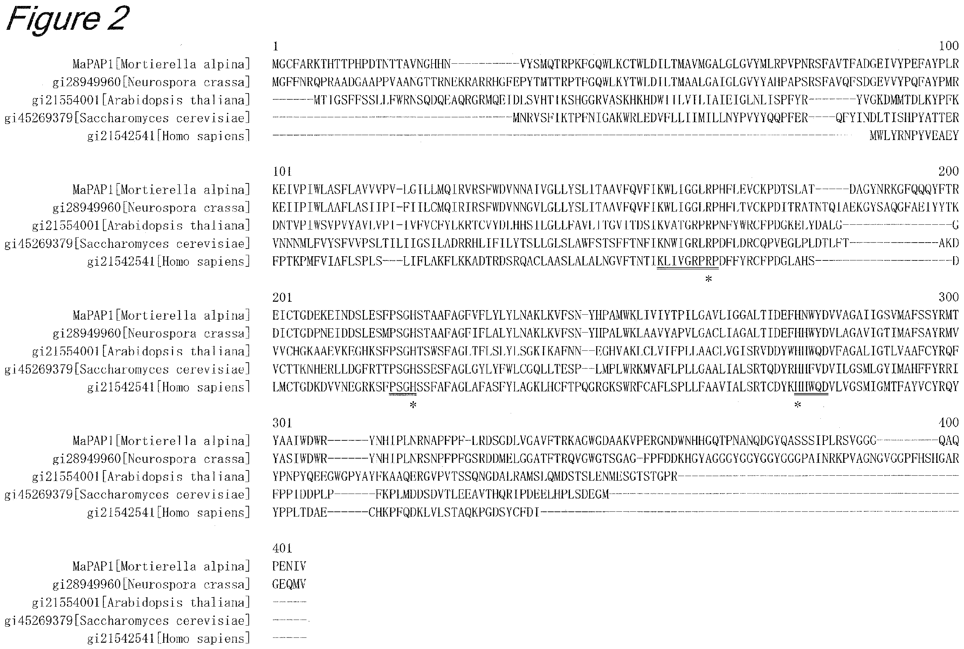

Those skilled in the art would be able to design and prepare appropriate mutants of the proteins described herein by using techniques known in the art. For example, when targeting a region which appears to be less important for the biological activity of the protein of the present invention, it is possible to identify a suitable region in the protein molecule whose structure can be changed without impairing the biological activity of the protein of the present invention. It is also possible to identify residues or regions in the molecule, which are conserved between similar proteins. Moreover, it is also possible to introduce conservative amino acid substitutions into a region which appears to be important for the biological activity or structure of the protein of the present invention, without impairing the biological activity and without adversely affecting the polypeptide structure of the protein. Particularly in the present invention, as double underlined in Figure 2, the amino acid sequence (SEQ ID NO: 2) of MaPAP1 of the present invention contains, at residues 146-154, 202-205 and 260-264, three consensus regions conserved among Mg²⁺-independent phosphatidic acid phosphatase 2 (PAP2) family enzymes. In these three consensus regions conserved among PAP2 family enzymes, arginine in domain 1 and histidines in domains 2 and 3 are known as amino acids essential for activity, and these amino acids are also conserved in MaPAP1 of the present invention as arginine at residue 153 and histidines at residues 205 and 260 of SEQ ID NO: 2. The above consensus regions are essential for PAP2 family enzymes and are also important for the PAP of the present invention. Thus, mutants according to the present invention are not limited in any way as long as the above consensus regions are conserved.

Those skilled in the art would be able to conduct a so-called structure-function study which identifies residues, in the protein of the present invention and in a similar peptide thereof, that are important for biological activity or structure, and compares amino acid residues between these two peptides, thereby predicting which residues in the protein similar to the protein of the present invention are amino acid residues corresponding to those important for biological activity or structure. Moreover, chemically similar amino acid substitutions may be chosen for the amino acid residues thus predicted to thereby select a mutant which retains the biological activity of the protein of the present invention. Likewise, those skilled in the art would also be able to analyze the three-dimensional structure and amino acid sequence of this protein mutant. The analysis results thus obtained can further be used to predict the alignment of amino acid residues with respect to the three-dimensional structure of the protein. Since amino acid residues predicted to be on the protein surface may be involved in important interactions with other molecules, those skilled in the art would be able to prepare a mutant which causes no change in these amino acid residues predicted to be on the protein surface, on the basis of analysis results as mentioned above. Moreover, those skilled in the art would also be able to prepare a mutant having a single amino acid substitution for any of the amino acid residues constituting the protein of the present invention. These mutants may be screened by any known assay to collect information about the individual mutants, which in turn allows evaluation of the usefulness of individual amino acid residues constituting the protein of the present invention when a comparison is made with the following case where a mutant having substitution of a specific amino acid residue shows lower biological activity than that of the protein of the present invention, where such a mutant shows no biological activity, or where such a mutant produces unsuitable activity to inhibit the biological activity of the protein of the present invention. Moreover, based on information collected from such routine experiments, those skilled in the art may readily analyze amino acid substitutions undesirable for mutants of the protein of the present invention either alone or in combination with other mutations.

As described above, a protein consisting of an amino acid sequence with deletion, substitution or addition of one or more amino acids in the amino acid sequence shown in SEQ ID NO: 2 can be prepared according to techniques such as site-directed mutagenesis as described in "Molecular Cloning, A Laboratory Manual 3rd ed." (Cold Spring Harbor Press (2001)), "Current Protocols in Molecular Biology" (John Wiley & Sons (1987-1997), Kunkel (1985) Proc. Natl. Acad. Sci. USA 82: 488-92, and Kunkel (1988) Method. Enzymol. 85: 2763-6. Preparation of a mutant with such a mutation including amino acid deletion, substitution or addition may be accomplished, for example, by known procedures such as Kunkel method or Gapped duplex method using a mutation-introducing kit based on site-directed mutagenesis such as a QuikChange^{™} Site-Directed Mutagenesis Kit (Stratagene), a GeneTailor^{™} Site-Directed Mutagenesis System (Invitrogen) or a TaKaRa Site-Directed Mutagenesis System (e.g., Mutan-K, Mutan-Super Express Km; Takara Bio Inc., Japan).

Techniques for allowing deletion, substitution or addition of one or more amino acids in the amino acid sequences of proteins while retaining their activity include site-directed mutagenesis mentioned above, as well as other techniques such as those for treating a gene with a mutagen, and those in which a gene is selectively cleaved to remove, substitute or add a selected nucleotide or nucleotides, and then ligated.

A preferred nucleotide sequence contained in the nucleic acids of the present invention is a nucleotide sequence which encodes a protein consisting of an amino acid sequence with deletion, substitution or addition of 1 to 10 amino acids in the amino acid sequence shown in SEQ ID NO: 2 and having PAP activity.

Moreover, nucleotide sequences contained in the nucleic acids of the present invention also preferably include a nucleotide sequence which encodes a protein consisting of an amino acid sequence with deletion, substitution or addition of 1 to 10 amino acids in SEQ ID NO: 2 and having the above activity of the present invention.

There is no limitation on the number or sites of amino acid mutations or modifications in the protein of the present invention, as long as the resulting mutant retains PAP activity or the ability to yield the fatty acid rate of PAP in the present invention.

PAP activity in the present invention or the ability to yield the fatty acid rate of PAP in the present invention can be measured in a known manner. For example, reference may be made to the following document: J. Biol. Chem., 273, 14331-14338 (1998).

"PAP activity" in the present invention may be measured as follows, by way of example. A microsomal fraction is prepared from yeast cells transformed to express the PAP of the present invention, as described in, e.g., J. Bacteriology, 173, 2026-2034 (1991). To a reaction solution containing 0.5 mM phosphatidic acid and 10 mM 2-mercaptoethanol in 50 mM Tris-HCl (pH 7.5), the above microsomal fraction is then added and reacted at 28°C for an appropriate period. Chloroform:methanol is added to stop the reaction, followed by lipid extraction. The resulting lipids are fractionated by thin-layer chromatography or other techniques, whereby the amount of diacylglycerol generated can be quantified.

Likewise, "the ability to yield the fatty acid rate of PAP" in the present invention may be measured as follows, by way of example. To lyophilized cells obtained by the method of the present invention for preparing a fatty acid composition, chloroform:methanol adjusted to an appropriate ratio is added and stirred, followed by heat treatment for an appropriate period. Centrifugation is further performed to separate the cells and collect the solvent. This procedure is repeated several times. Then, lipids are dried up in an appropriate manner, and a solvent such as chloroform is added to dissolve the lipids. An appropriate aliquot of this sample is treated by the hydrochloric acid/methanol method to derive fatty acids in the cells into corresponding methyl esters, followed by extraction with hexane. After distilling off hexane, the fatty acids are analyzed by gas chromatography.

(b) A nucleotide sequence which is hybridizable under stringent conditions with a nucleic acid consisting of a nucleotide sequence complementary to a nucleotide sequence consisting of SEQ ID NO: 4 and which encodes a protein having the above activity of the present invention.

Nucleotide sequences contained in the nucleic acids of the present invention include a nucleotide sequence which is hybridizable under stringent conditions with a nucleic acid consisting of a nucleotide sequence complementary to a nucleotide sequence consisting of SEQ ID NO: 4 and which encodes a protein having the above activity of the present invention. SEQ ID NO: 4 and PAP activity are as described above.

To obtain the above nucleotide sequence, a probe may be prepared from an appropriate fragment in a manner known to those skilled in the art, and this probe may be used in known hybridization techniques such as colony hybridization, plaque hybridization or Southern blotting to obtain the nucleotide sequence from a cDNA library, a genomic library or the like.

As to detailed procedures for hybridization techniques, reference may be made to "Molecular Cloning, A Laboratory Manual 3rd ed." (Cold Spring Harbor Press (2001); particularly Sections 6-7), "Current Protocols in Molecular Biology" (John Wiley & Sons (1987-1997); particularly Sections 6.3-6.4), "DNA Cloning 1: Core Techniques, A Practical Approach 2nd ed." (Oxford University (1995); particularly Section 2.10 for hybridization conditions).

The strength of hybridization is determined primarily by hybridization conditions, more preferably by hybridization conditions and washing conditions. The term "stringent conditions" as used herein is intended to include moderately or highly stringent conditions.

More specifically, moderately stringent conditions include, for example, hybridization conditions of 1 × SSC to 6 × SSC at 42°C to 55°C, more preferably 1 × SSC to 3 × SSC at 45°C to 50°C, and most preferably 2 × SSC at 50°C. In certain cases such as where a hybridization solution contains about 50% formamide, a temperature which is 5°C to 15°C lower than the above temperature is used. Washing conditions may be 0.5 × SSC to 6 × SSC at 40°C to 60°C. During hybridization and washing, 0.05% to 0.2% SDS, preferably about 0.1% SDS may usually be added.

Highly stringent (high stringent) conditions include hybridization and/or washing at higher temperature and/or lower salt concentration, compared to the moderately stringent conditions. For example, hybridization conditions may be 0.1 × SSC to 2 × SSC at 55°C to 65°C, more preferably 0.1 × SSC to 1 × SSC at 60°C to 65°C, and most preferably 0.2 × SSC at 63°C. Washing conditions may be 0.2 × SSC to 2 × SSC at 50°C to 68°C, and more preferably 0.2 × SSC at 60°C to 65°C.

Hybridization conditions particularly used in the present invention include, but are not limited to, prehybridization in 5 × SSC, 1% SDS, 50 mM Tris-HCl (pH 7.5) and 50% formamide at 42°C, overnight incubation at 42°C in the presence of a probe to form hybrids, and the subsequent three washings in 0.2 × SSC, 0.1% SDS at 65°C for 20 minutes.

It is also possible to use a commercially available hybridization kit which uses no radioactive substance as a probe. Specific examples include hybridization with a DIG nucleic acid detection kit (Roche Diagnostics) or with an ECL direct labeling & detection system (Amersham).

(c) A nucleotide sequence which consists of a nucleotide sequence sharing an identity of 85% or more with a nucleotide sequence consisting of SEQ ID NO: 4 and which encodes a protein having the above activity of the present invention.

Nucleotide sequences contained in the nucleic acids of the present invention include a nucleotide sequence which consists of a nucleotide sequence sharing an identity of at least 85% or more with the nucleic acid sequence shown in SEQ ID NO: 4 and which encodes a protein having the above activity of the present invention.

Preferred examples include nucleic acids comprising a nucleotide sequence which shares an identity of at least 85% or more, even more preferably 90% or more, more particularly 95%, 98% or 99%) with the nucleic acid sequence shown in SEQ ID NO: 4 and which encodes a protein having the above activity of the present invention.

The percent identity between two nucleic acid sequences can be determined by visual inspection and mathematical calculation, or more preferably by using a computer program to compare sequence information between two nucleic acids. Computer programs for sequence comparison include, for example, the BLASTN program (Altschul et al. (1990) J. Mol. Biol. 215: 403-10) version 2.2.7, available for use via the National Library of Medicine website: http://www.ncbi.nlm.nih.gov/blast/bl2seq/bls.html, or the WU-BLAST 2.0 algorithm. Standard default parameter settings for WU-BLAST 2.0 are described at the following Internet site: http://blast.wustl.edu.

(d) A nucleotide sequence which encodes an amino acid sequence sharing an identity of 90% or more with an amino acid sequence consisting of SEQ ID NO: 2 and which encodes a protein having the above activity of the present invention.

Nucleotide sequences contained in the nucleic acids of the present invention include a nucleotide sequence which encodes an amino acid sequence sharing an identity of 90% or more with an amino acid sequence consisting of SEQ ID NO: 2 and which encodes a protein having the above activity of the present invention. Proteins encoded by the nucleic acids of the present invention may also be those sharing identity with the amino acid sequence of MaPAP1, as long as they are functionally equivalent to proteins having the above activity of the present invention.

Specific examples include amino acid sequences sharing an identity of 90% (e.g., 95%, more particularly 98%) with the amino acid sequence shown in SEQ ID NO: 2.

A preferred nucleotide sequence contained in the nucleic acids of the present invention is a nucleotide sequence which encodes an amino acid sequence sharing an identity of 90% or more with an amino acid sequence consisting of SEQ ID NO: 2 and which encodes a protein having the above activity of the present invention. More preferred is a nucleotide sequence which encodes an amino acid sequence sharing an identity of 95% or more with an amino acid sequence consisting of SEQ ID NO: 2 and which encodes a protein having the above activity of the present invention.

The percent identity between two amino acid sequences may be determined by visual inspection and mathematical calculation. Alternatively, the percent identity may be determined by using a computer program. Examples of such a computer program include BLAST, FASTA (Altschul et al., J. Mol. Biol., 215:403-410 (1990)) and ClustalW. In particular, various conditions (parameters) for an identity search with the BLAST program are described by Altschul et al. (Nucl. Acids. Res., 25, p.3389-3402, 1997) and publicly available via the website of the National Center for Biotechnology Information (NCBI) or the DNA Data Bank of Japan (DDBJ) (BLAST Manual, Altschul et al., NCB/NLM/NIH Bethesda, MD 20894; Altschul et al.). It is also possible to use a program such as genetic information processing software GENETYX Ver.7 (Genetyx Corporation, Japan), DINASIS Pro (Hitachisoft, Japan) or Vector NTI (Infomax) for determination of the percent identity.

Certain alignment schemes for aligning amino acid sequences may also result in matching of a specific short region of the sequences, and it is also possible to detect a region with very high sequence identity in such a small aligned region even when there is no significant relationship between the full-length sequences used. In addition, the BLAST algorithm uses the BLOSUM62 amino acid scoring matrix, and optional parameters that can be used are as follows: (A) inclusion of a filter to mask segments of the query sequence that have low compositional complexity (as determined by the SEG program of Wootton and Federhen (Computers and Chemistry, 1993); also see Wootton and Federhen, 1996, "Analysis of compositionally biased regions in sequence databases," Methods Enzymol., 266: 554-71) or segments consisting of short-periodicity internal repeats (as determined by the XNU program of Claverie and States (Computers and Chemistry, 1993)), and (B) a statistical significance threshold for reporting matches against database sequences, or E-score (the expected probability of matches being found merely by chance, according to the stochastic model of Karlin and Altschul, 1990; if the statistical significance ascribed to a match is greater than this E-score threshold, the match will not be reported).

Preferred embodiments for the nucleic acids of the present invention also include a nucleic acid comprising a nucleotide sequence shown in any one of (a) to (c) below or a fragment thereof:
(a) the nucleotide sequence shown in SEQ ID NO: 4;
(b) a nucleotide sequence encoding a protein consisting of the amino acid sequence shown in SEQ ID NO: 2; or
(c) the nucleotide sequence shown in SEQ ID NO: 1.

The above (a) nucleotide sequence shown in SEQ ID NO: 4, (b) nucleotide sequence encoding a protein consisting of the amino acid sequence shown in SEQ ID NO: 2, and (c) nucleotide sequence shown in SEQ ID NO: 1 are as shown in Table 1. Fragments of these sequences may be either naturally occurring or artificially prepared, including regions contained in the above nucleotide sequences, i.e., ORF, CDS, a biologically active region, a region used as a primer as described later, and a region which may serve as a probe.

### Phosphatidic acid phosphatase proteins of the present invention

The proteins of the present invention, which may be either naturally occurring or artificially prepared, include a protein consisting of the amino acid sequence shown in SEQ ID NO: 2 and proteins functionally equivalent to this protein. Such a protein consisting of the amino acid sequence shown in SEQ ID NO: 2 is as described above. "Proteins functionally equivalent" are intended to mean proteins having "the above activity of the present invention," as explained in the section "Nucleic acids of the present invention encoding phosphatidic acid phosphatase" described above.

In the present invention, proteins functionally equivalent to a protein consisting of the amino acid sequence shown in SEQ ID NO: 2 include a protein shown in (a) or (b) below:
(a) a protein which consists of an amino acid sequence with deletion, substitution or addition of one or more amino acids in SEQ ID NO: 2 and which has the above activity of the present invention; or
(b) a protein which consists of an amino acid sequence sharing an identity of 85% or more with an amino acid sequence consisting of SEQ ID NO: 2 and which has the above activity of the present invention.

Among the above, the amino acid sequence with deletion, substitution or addition of one or more amino acids in SEQ ID NO: 2 or the amino acid sequence sharing an identity of 85% or more with an amino acid sequence consisting of SEQ ID NO: 2 is as explained in the section "Nucleic acids of the present invention encoding phosphatidic acid phosphatase" described above. The phrase "protein which has the above activity of the present invention" is intended to also include mutants of a protein encoded by a nucleic acid comprising the nucleotide sequence of SEQ ID NO: 4, or mutated proteins with various modifications such as substitution, deletion or addition of one or more amino acids in the amino acid sequence shown in SEQ ID NO: 2, as well as their modified proteins whose amino acid side chains or the like are modified, and their fusion proteins with other proteins, as long as these proteins have PAP activity and/or the ability to yield the fatty acid rate of PAP in the present invention.

The proteins of the present invention may also be artificially prepared by chemical synthesis techniques such as Fmoc method (fluorenylmethyloxycarbonyl method) and tBoc method (t-butyloxycarbonyl method). In addition, peptide synthesizers available from Advanced ChemTech, Perkin Elmer, Pharmacia, Protein Technology Instrument, Synthecell-Vega, PerSeptive, Shimadzu Corporation (Japan) or other manufacturers may be used for chemical synthesis.

### Cloning of PAP nucleic acids

The PAP nucleic acids of the present invention can be cloned, for example, by screening from a cDNA library using an appropriate probe. They can also be cloned by PCR amplification with appropriate primers and the subsequent ligation to an appropriate vector. The clones thus obtained may further be subcloned into another vector.

For example, it is possible to use commercially available plasmid vectors including pBlue-Script^{™} SK(+) (Stratagene), pGEM-T (Promega), pAmp (TM: Gibco-BRL), p-Direct (Clontech) and pCR2.1-TOPO (Invitrogen). In the case of using PCR amplification, primers may be any regions of the nucleotide sequence shown in, e.g., SEQ ID NO: 1. By way of example, it is possible to use the following primers from SEQ ID NO: 1:
D-1: 5'-CATGGGTTGCTTCGCGCGCAAGACG-3' (SEQ ID NO: 5) as an upstream primer; and
D-2: 5'-CGAAGCCGGCAAAGGCGGCAGTC-3' (SEQ ID NO: 6) as a downstream primer. Then, PCR is performed on cDNA prepared from *M. alpina* cells with the above primers and DNA polymerase or the like. Although this procedure can be readily accomplished by those skilled in the art according to, e.g., "Molecular Cloning, A Laboratory Manual 3rd ed." (Cold Spring Harbor Press (2001)), PCR conditions in the present invention may be set as follows, by way of example:
   Denaturation temperature: 90-95°C
   Annealing temperature: 40-60°C
   Elongation temperature: 60-75°C
   Number of cycles: 10 or more cycles.

The resulting PCR products may be purified in a known manner, for example, by using a kit (e.g., GENECLEAN (Funakoshi Co., Ltd., Japan), QIAquick PCR purification Kits (QIAGEN), ExoSAP-IT (GE Healthcare Bio-Sciences)), a DEAE-cellulose filter or a dialysis tube. In the case of using an agarose gel, the PCR products are subjected to agarose gel electrophoresis and nucleotide sequence fragments are excised from the agarose gel, followed by purification with GENECLEAN (Funakoshi Co., Ltd., Japan) or QIAquick Gel extraction Kits (QIAGEN) or by the freeze-squeeze method, etc.

The cloned nucleic acids can be determined for their nucleotide sequences with a nucleotide sequencer.

### Vector construction for PAP expression and transformant preparation

The present invention also provides a recombinant vector comprising a nucleic acid encoding the PAP of the present invention. The present invention further provides a transformant transformed with the above recombinant vector.

Such a recombinant vector and transformant can be obtained as follows. Namely, a plasmid carrying a nucleic acid encoding the PAP of the present invention is digested with restriction enzymes. Examples of restriction enzymes available for use include, but are not limited to, EcoRI, KpnI, BamHI and SalI. This digestion may be followed by blunt ending with T4 polymerase. The digested nucleotide sequence fragment is purified by agarose gel electrophoresis. This nucleotide sequence fragment may be integrated into an expression vector in a known manner to obtain a vector for PAP expression. This expression vector is introduced into a host to prepare a transformant, which is then provided for expression of a desired protein.

In this case, the types of expression vector and host are not limited in any way as long as they allow expression of a desired protein. Examples of a host include fungi, bacteria, plants, animals or cells thereof. Fungi include filamentous fungi such as lipid-producing *M. alpina,* and yeast strains such as *Saccharomyces cerevisiae.* Bacteria include *Escherichia coli (E. coli)* and *Bacillus subtilis.* Likewise, plants include oil plants such as rapeseed, soybean, cotton, safflower and flax.

As lipid-producing strains, those such as found in MYCOTAXON, Vol. XLIV, NO. 2, pp. 257-265 (1992) can be used. Specific examples include microorganisms belonging to the genus *Mortierella,* as exemplified by microorganisms belonging to the subgenus *Mortierella* such as *Mortierella elongata* IFO8570, *Mortierella exigua* IFO8571, *Mortierella hygrophila* IFO5941, *Mortierella alpina* IFO8568, ATCC16266, ATCC32221, ATCC42430, CBS 219.35, CBS224.37, CBS250.53, CBS343.66, CBS527.72, CBS528.72, CBS529.72, CBS608.70, CBS754.68, as well as microorganisms belonging to the subgenus *Micromucor* such as *Mortierella isabellina* CBS 194.28, IFO6336, IFO7824, IFO7873, IFO7874, IFO8286, IFO8308, IFO7884, *Mortierella nana* IFO8190, *Mortierella ramanniana* IFO5426, IFO8186, CBS112.08, CBS212.72, IFO7825, IFO8184, IFO8185, IFO8287, *Mortierella vinacea* CBS236.82. Particularly preferred is *Mortierella alpina.*

When a fungus is used as a host, it is desirable that the nucleic acid of the present invention is self-replicable in the host or has a structure insertable onto the fungal chromosome. At the same time, it is preferable to further comprise a promoter and a terminator. When *M. alpina* is used as a host, examples of an expression vector include pD4, pDuraSC and pDura5. Any promoter may be used as long as it allows expression in the host, and examples include promoters derived from *M. alpina,* such as histonH4.1 gene promoter, GAPDH (glyceraldehyde-3-phosphate dehydrogenase) gene promoter and TEF (translation elongation factor) gene promoter.

Techniques for introducing a recombinant vector into filamentous fungi (e.g., *M. alpina*) include electroporation, spheroplast and particle delivery methods, as well as direct microinjection of DNA into nuclei. In the case of using an auxotrophic host strain, strains growing on a selective medium lacking nutrients required for the host strain may be selected to thereby obtain transformed strains. Alternatively, in a case where a drug resistance marker gene is used for transformation, culture may be carried out with a selective medium containing the drug to thereby obtain cell colonies resistant to the drug.

When yeast is used as a host, examples of an expression vector include pYE22m. Alternatively, commercially available yeast expression vectors such as pYES (Invitrogen) and pESC (STRATAGENE) may also be used. Yeast hosts suitable for the present invention include, but are not limited to, *Saccharomyces cerevisiae* strain EH13-15 (trp1, MATα). Examples of a promoter available for use include those derived from yeast or the like, such as GAPDH promoter, gal1 promoter and gal10 promoter.

Techniques for introducing a recombinant vector into yeast cells include lithium acetate, electroporation and spheroplast methods, as well as dextran-mediated transfection, calcium phosphate precipitation, polybrene-mediated transfection, protoplast fusion, encapsulation of polynucleotide(s) in liposomes, and direct microinjection of DNA into nuclei.

When a bacterium such as *E. coli* is used as a host, examples of an expression vector include pGEX and pUC18 available from Pharmacia. Examples of a promoter available for use include those derived from *E. coli,* phage or the like, such as trp promoter, lac promoter, PL promoter and PR promoter. Techniques for introducing a recombinant vector into bacteria include electroporation and calcium chloride methods.

### Method of the present invention for preparing a fatty acid composition

The present invention provides a method for preparing a fatty acid composition from the above transformant, i.e., a method for preparing a fatty acid composition from a cultured product obtained by culturing the above transformant, more specifically as described below. However, the method of the present invention is not limited to the following, and may be accomplished in any other manner generally known.

For culture of organisms transformed to express PAP, any medium may be used as long as it is a culture solution (medium) having appropriate pH and osmotic pressure as well as containing nutrients required for growth of each host, trace elements, and biomaterials such as serum or antibiotics. For example, in the case of yeast cells transformed to express PAP, SC-Trp medium, YPD medium, YPD5 medium or the like may be used without being limited thereto. Detailed medium composition is illustrated for SC-Trp medium: 6.7 g Yeast nitrogen base w/o amino acids (DIFCO), 20 g glucose and 1.3 g amino acid powder (a mixture of 1.25 g adenine sulfate, 0.6 g arginine, 3 g aspartic acid, 3 g glutamic acid, 0.6 g histidine, 1.8 g leucine, 0.9 g lysine, 0.6 g methionine, 1.5 g phenylalanine, 11.25 g serine, 0.9 g tyrosine, 4.5 g valine, 6 g threonine and 0.6 g uracil) per liter of medium.

Any culture conditions may be used as long as they are suitable for host growth and are adequate for maintenance of the generated enzyme in a stable state. More specifically, individual conditions may be adjusted, including anaerobic degree, culture period, temperature, humidity, static culture or shaking culture. Culture may be accomplished under the same conditions (one-step culture) or by so-called two-step or three-step culture using two or more different culture conditions. For large-scale culture, two-step or more step culture is preferred because of its high culture efficiency.

To explain detailed procedures for the method of the present invention for preparing a fatty acid composition, two-step culture in which yeast is used as a host will be illustrated below as an example. Namely, in the pre-culture step, the colonies obtained above are inoculated into any medium described above (e.g., SC-Trp medium) and cultured with shaking at 30°C for 2 days. Then, in the main culture step, the pre-cultured solution (500 µl) is added to 10 ml YPD5 (2% yeast extract, 1% polypeptone, 5% glucose) medium and cultured with shaking at 30°C for 2 days.

### Fatty acid compositions of the present invention

The present invention also provides a fatty acid composition which is a collection of one or more fatty acids in cells expressing the PAP of the present invention. Fatty acids may be free fatty acids or may be triglycerides, phospholipids or the like. In particular, the fatty acid composition of the present invention is characterized by having a fatty acid rate ensuring a higher ratio of at least one or more of:
i) the oleic acid content;
ii) the ratio of the palmitoleic acid content to the palmitic acid content;
iii) the ratio of the oleic acid content to the palmitic acid content;
iv) the ratio of the total content of stearic acid and oleic acid to the palmitic acid content;
v) the ratio of the C₁₈ fatty acid content to the C₁₆ fatty acid content; and
vi) the ratio of the arachidonic acid, dihomo-γ-linolenic acid and/or γ-linolenic acid content
when compared to a cultured product obtained by culturing a host which is not transformed with the recombinant vector of the present invention. The phrase "host which is not transformed with the recombinant vector of the present invention" as used herein is intended to mean, for example, a host transformed with an empty vector carrying none of the nucleic acids described in the section "Nucleic acids of the present invention encoding phosphatidic acid phosphatase."

Fatty acids contained in the fatty acid composition of the present invention refer to linear or branched monocarboxylic acids of long-chain carbohydrates, including but not limited to, myristic acid (tetradecanoic acid) (14:0), myristoleic acid (tetradecenoic acid) (14:1), palmitic acid (hexadecanoic acid) (16:0), palmitoleic acid (9-hexadecenoic acid) (16:1), stearic acid (octadecanoic acid) (18:0), oleic acid (cis-9-octadecenoic acid) (18:1(9)), vaccenic acid (11-octadecenoic acid) (18:1(11)), linolic acid (cis,cis-9,12 octadecadienoic acid) (18:2(9,12)), α-linolenic acid (9,12,15-octadecatrienoic acid) (18:3(9,12,15)), γ-linolenic acid (6,9,12-octadecatrienoic acid) (18:3(6,9,12)), stearidonic acid (6,9,12,15-octadecatetraenoic acid) (18:4(6,9,12,15)), arachidic acid (icosanoic acid) (20:0), (8,11-icosadienoic acid) (20:2(8,11)), mead acid (5,8,11-icosatrienoic acid) (20:3(5,8,11)), dihomo-γ-linolenic acid (8,11,14-icosatrienoic acid) (20:3(8,11,14)), arachidonic acid (5,8,11,14-icosatetraenoic acid) (20:4(5,8,11,14)), eicosatetraenoic acid (8,11,14,17-icosatetraenoic acid) (20:4(8,11,14,17)), eicosapentaenoic acid (5,8,11,14,17-icosapentaenoic acid) (20:5(5,8,11,14,17)), behenic acid (docosanoic acid) (22:0), (7,10,13,16-docosatetraenoic acid) (22:4(7,10,13,16)), (7,10,13,16,19-docosapentaenoic acid) (22:5(7,10,13,16,19)), (4,7,10,13,16-docosapentaenoic acid) (22:5(4,7,10,13,16)), (4,7,10,13,16,19-docosahexaenoic acid) (22:6(4,7,10,13,16,19)), lignoceric acid (tetradocosanoic acid) (24:0), nervonic acid (cis-15-tetradocosanoic acid) (24:1) and cerotic acid (hexadocosanoic acid) (26:0). It should be noted that the above substance names are common names defined by the IUPAC Biochemical Nomenclature, and their systematic names are given in parentheses along with numerics denoting the number of carbons and the positions of double bonds.

The fatty acid composition of the present invention may be composed of any number and any type of fatty acids, as long as it is a combination of one or more fatty acids selected from those listed above.

Whether such a fatty acid composition of the present invention is obtained, i.e., whether the PAP of the present invention is expressed may be confirmed in a manner generally known, for example, as a change in fatty acid rate when PAP is expressed in yeast cells. Namely, to lyophilized cells obtained by the above method of the present invention for preparing a fatty acid composition, chloroform:methanol adjusted to an appropriate ratio is added and stirred, followed by heat treatment for an appropriate period. Centrifugation is further performed to separate the cells and collect the solvent. This procedure is repeated several times. Then, lipids are dried up in an appropriate manner, and a solvent such as chloroform is added to dissolve the lipids. An appropriate aliquot of this sample is treated by the hydrochloric acid/methanol method to derive fatty acids in the cells into corresponding methyl esters, followed by extraction with hexane. After distilling off hexane, the fatty acids are analyzed by gas chromatography.

As a result, if a fatty acid composition having the above fatty acid rate is obtained, it can be determined that the fatty acid composition of the present invention was obtained.

Food or other products comprising fatty acid compositions of the present invention

The present invention also provides a food product comprising the above fatty acid composition. The fatty acid composition of the present invention can be used in a routine manner for purposes such as production of food products containing fats and oils as well as production of industrial source materials (those for cosmetics, pharmaceuticals (e.g., external preparations for skin), soaps, etc.). Cosmetics (cosmetic compositions) or pharmaceuticals (pharmaceutical compositions) may be formulated into any dosage form including, but not limited to, solutions, pastes, gels, solids or powders. Likewise, possible forms of food products include pharmaceutical formulations such as capsules, as well as processed foods such as ordinary fluid diets, semi-digested nourishing diets, elemental diets, drinkable preparations or enteral nutrient preparations, which comprise the fatty acid composition of the present invention in admixture with proteins, sugars, fats, trace elements, vitamins, emulsifiers, flavorings, etc.

Moreover, examples of the food product of the present invention include, but are not limited to, nutritional supplementary foods, health foods, functional foods, children's foods, infant modified milk, premature infant modified milk, and geriatric foods. The term "food" or "food product" is used herein as a generic name for edible materials in the form of solids, fluids, liquids or mixtures thereof.

The term "nutritional supplementary foods" refers to food products enriched with specific nutritional ingredients. The term "health foods" refers to food products that are healthful or good for health, and encompasses nutritional supplementary foods, natural foods and diet foods. The term "functional foods" refers to food products for replenishing nutritional ingredients which assist body control functions. Functional foods are synonymous with foods for specified health use. The term "children's foods" refers to food products given to children up to about 6 years old. The term "geriatric foods" refers to food products treated to facilitate digestion and absorption when compared to untreated foods. The term "infant modified milk" refers to modified milk given to children up to about one year old. The term "premature infant modified milk" refers to modified milk given to premature infants until about 6 months after birth.

These food products include natural foods (treated with fats and oils) such as meat, fish and nuts; foods supplemented with fats and oils during preparation (e.g., Chinese foods, Chinese noodles, soups); foods prepared using fats and oils as heating media (e.g., tempura (deep-fried fish and vegetables), deep-fried foods, fried bean curd, Chinese fried rice, doughnuts, Japanese fried dough cookies (karinto)); fat- and oil-based foods or processed foods supplemented with fats and oils during processing (e.g., butter, margarine, mayonnaise, dressing, chocolate, instant noodles, caramel, biscuits, cookies, cake, ice cream); and foods sprayed or coated with fats and oils upon finishing (e.g., rice crackers, hard biscuits, sweet bean paste bread). However, the food product of the present invention is not limited to foods containing fats and oils, and other examples include agricultural foods such as bakery products, noodles, cooked rice, sweets (e.g., candies, chewing gums, gummies, tablets, Japanese sweets), bean curd and processed products thereof; fermented foods such as Japanese rice wine (sake), medicinal liquor, sweet cooking sherry (mirin), vinegar, soy sauce and miso (bean paste); livestock food products such as yogurt, ham, bacon and sausage; seafood products such as fish cake (kamaboko), deep-fried fish cake (ageten) and puffy fish cake (hanpen); as well as fruit drinks, soft drinks, sports drinks, alcoholic beverages, and tea.

Method for strain evaluation or selection using PAP-encoding nucleic acid or PAP protein of the present invention

The present invention also provides a method for evaluating or selecting a lipid-producing strain using the PAP-encoding nucleic acid or PAP protein of the present invention. Details are given below.

### (1) Evaluation method

One embodiment of the present invention is a method for evaluating a lipid-producing strain using the PAP-encoding nucleic acid or PAP protein of the present invention. As a first example for the above evaluation method of the present invention, lipid-producing test strains are evaluated for the above activity of the present invention by using primers or probes designed based on the nucleotide sequence of the present invention. General procedures for such evaluation are known and can be found in, e.g., International Patent Publication No. WO01/040514 or JP 8-205900 A. A brief explanation will be given below of this evaluation.

First, the genome of a test strain is prepared. For genome preparation, it is possible to use any known technique such as Hereford method or potassium acetate method (see, e.g., Methods in Yeast Genetics, Cold Spring Harbor Laboratory Press, p130 (1990)).

Primers or probes are designed based on the nucleotide sequence of the present invention, preferably SEQ ID NO: 4. These primers or probes may be any regions of the nucleotide sequence of the present invention, and known procedures may be used for their design. The number of nucleotides in a polynucleotide used as a primer is generally 10 nucleotides or more, preferably 15 to 25 nucleotides. Likewise, the number of nucleotides appropriate for a region to be flanked by primers is generally 300 to 2000 nucleotides.

The primers or probes prepared above are used to examine whether the genome of the above test strain contains a sequence specific to the nucleotide sequence of the present invention. A sequence specific to the nucleotide sequence of the present invention may be detected using known procedures. For example, a polynucleotide comprising a part or all of a sequence specific to the nucleotide sequence of the present invention or a polynucleotide comprising a nucleotide sequence complementary to the above nucleotide sequence is used as one primer, and a polynucleotide comprising a part or all of a sequence located upstream or downstream of this sequence or a polynucleotide comprising a nucleotide sequence complementary to the above nucleotide sequence is used as the other primer to amplify nucleic acids from the test strain by PCR or other techniques, followed by determining the presence or absence of amplification products, the molecular weight of amplification products, etc.

PCR conditions suitable for the method of the present invention are not limited in any way, and may be set as follows, by way of example:
Denaturation temperature: 90-95°C
Annealing temperature: 40-60°C
Elongation temperature: 60-75°C
Number of cycles: 10 or more cycles.
The resulting reaction products may be separated by electrophoresis on an agarose gel or the like to determine the molecular weight of the amplification products. Each amplification product is then confirmed as to whether its molecular weight is a size enough to cover a nucleic acid molecule corresponding to a region specific to the nucleotide sequence of the present invention, whereby the test strain can be predicted or evaluated for the above activity of the present invention. Moreover, if the above amplification products are analyzed for their nucleotide sequences, as described above, the above activity of the present invention can be predicted or evaluated with more accuracy. It should be noted that procedures for evaluating the above activity of the present invention are as described above.

As another example for the above evaluation method of the present invention, a test strain is cultured and measured for the expression level of PAP encoded by the nucleotide sequence of the present invention (e.g., SEQ ID NO: 4), whereby the test strain can be evaluated for the above activity of the present invention. It should be noted that the expression level of PAP can be measured by culturing a test strain under appropriate conditions and quantifying mRNA or protein for PAP. Quantification of mRNA or protein may be accomplished by using known procedures, for example, Northern hybridization or quantitative RT-PCR for mRNA quantification and Western blotting for protein quantification (Current Protocols in Molecular Biology, John Wiley & Sons 1994-2003). For evaluation of the above activity, it is also possible to measure the fatty acid rate of a fatty acid composition produced by the PAP of the present invention. Procedures for measuring the fatty acid rate of a fatty acid composition are as described above.

### (2) Selection method

Another embodiment of the present invention is a method for selecting a lipid-producing strain using the PAP-encoding nucleic acid or PAP protein of the present invention. As an example for the above selection method of the present invention, test strains are cultured and measured for the expression level of PAP encoded by the nucleotide sequence of the present invention (e.g., SEQ ID NO: 4) to select a strain with a desired expression level, whereby a strain having a desired activity can be selected. Alternatively, a type strain is predetermined, and this type strain and test strains are each cultured and measured for the above expression level, followed by comparison of the expression level between the type strain and each test strain, whereby a desired strain can be selected. More specifically, for example, a type strain and test strains are cultured under appropriate conditions and measured for their expression levels to select a test strain showing higher or lower expression than the type strain, whereby a strain having a desired activity can be selected. Examples of a desired activity include the expression level of PAP and the fatty acid rate of a fatty acid composition produced by PAP, which may be measured as described above.

As another example for the above selection method of the present invention, test strains are cultured to select a strain in which the above activity of the present invention is high or low, whereby a strain having a desired activity can be selected. Examples of a desired activity include the expression level of PAP and the fatty acid rate of a fatty acid composition produced by PAP, which may be measured as described above.

Examples of a test strain or type strain available for use include, but are not limited to, a strain transformed with the above vector of the present invention, a strain modified to suppress expression of the above nucleic acid of the present invention, a strain modified by mutagenesis, and a strain having natural mutation(s). It should be noted that PAP activity in the present invention and the ability to yield the fatty acid rate of PAP in the present invention can be measured, for example, by the procedures described in the sections "Nucleic acids of the present invention encoding phosphatidic acid phosphatase" and "Fatty acid compositions of the present invention." Mutagenesis may be accomplished by, but not limited to, physical techniques including ultraviolet or radioactive irradiation, or chemical techniques including treatment with an agent such as EMS (ethylmethane sulfonate) or N-methyl-N-nitrosoguanidine (see, e.g., Yasuji Oshima ed., Biochemistry Experiments vol. 39, Experimental Protocols for Yeast Molecular Genetics, pp. 67-75, Japan Scientific Societies Press).

Strains used in the present invention as type and test strains include, but are not limited to, the above lipid-producing strains or yeast strains. More specifically, the type strain or test strain may be a combination of any strains belonging to different genera or species, and one or more test strains may be used simultaneously.

The present invention will now be described in more detail by way of the following examples.

### Example 1

### (1) EST analysis

*M. alpina* strain 1S-4 was inoculated into 100 ml medium (1.8% glucose, 1% yeast extract, pH 6.0) and pre-cultured for 3 days at 28°C. A 10 L culture vessel (Able Co., Tokyo) was charged with 5 L medium (1.8% glucose, 1% soybean powder, 0.1% olive oil, 0.01% Adekanol, 0.3% KH₂PO₄, 0.1% Na₂SO₄, 0.05% CaCl₂·2H₂O, 0.05% MgCl₂·6H₂O, pH 6.0) and inoculated with the entire pre-cultured product, followed by aerobic spinner culture under conditions of 300 rpm, 1 vvm and 26°C for 8 days. On days 1, 2 and 3 of culture, glucose was added in an amount corresponding to 2%, 2% and 1.5%, respectively. The cells were collected at each stage of culture (day 1, 2, 3, 6 or 8) to prepare total RNA by the guanidine hydrochloride/CsCl method. Using an Oligotex-dT30<Super>mRNA Purification Kit (Takara Bio Inc., Japan), poly(A)⁺RNA was purified from the total RNA. A cDNA library was prepared for each stage with a ZAP-cDNA Synthesis Kit (STRATAGENE), followed by one-pass sequence analysis from the 5'-end of cDNA (8000 clones × 5 stages). The resulting sequences were clustered.

### (2) Search for PAP gene homologs

The nucleotide sequences obtained from EST analysis were searched against amino acid sequences registered in GENBANK with a homology search program, BLASTX, to extract homologs of the PAP gene. As a result, among the ESTs, a sequence (SEQ ID NO: 1) was found that had high homology with a *Neurospora crassa*-derived putative protein which is a homolog of diacylglycerol pyrophosphate phosphatase (DPP) which has PAP activity. A contig obtained for this SEQ ID NO: 1 by EST analysis was composed of 9 sequences from the library on day 8 in (1) above.

### Example 2

### (1) Cloning of PAP homolog (MaPAP1)

To obtain a cDNA fragment containing the full-length ORF of PAP homolog, the following primers were designed.
Primer D-1 :CATGGGTTGCTTCGCGCGCAAGACG (SEQ ID NO: 5)
Primer D-2 :CGAAGCCGGCAAAGGCGGCAGTC (SEQ ID NO: 6)
Using these primers, PCR was performed with ExTaq (Takara Bio Inc., Japan) by using the cDNA library on day 8 as a template. The resulting DNA fragment of 0.64 kbp was TA-cloned with a TOPO-TA cloning Kit (Invitrogen) to determine the nucleotide sequence of an insert.

The results confirmed that a DNA fragment comprising a nucleotide sequence covering nucleotides 113-744 of SEQ ID NO: 1 was cloned. This plasmid was designated as pCR-2051-P. Then, the plasmid pCR-2051-P was used as a template to perform PCR with the same primers as shown above. In PCR, ExTaq (Takara Bio Inc., Japan) was used, but the attached dNTP mix was replaced by a PCR labeling mix (Roche Diagnostics) for digoxigenin (DIG) labeling of DNA to be amplified, thereby preparing a probe for use in cDNA library screening. This probe was used to screen the cDNA library on day 8.

Hybridization conditions were set as follows.
Buffer: 5 × SSC, 1% SDS, 50 mM Tris-HCl (pH 7.5), 50% formamide
Temperature: 42°C (overnight)
Washing: in 0.2 × SSC, 0.1% SDS at 65°C for 20 minutes (repeated three times)

Detection was accomplished by using a DIG nucleic acid detection kit (Roche Diagnostics). From phage clones obtained by screening, the plasmid was excised by in vivo excision to obtain plasmid DNA.

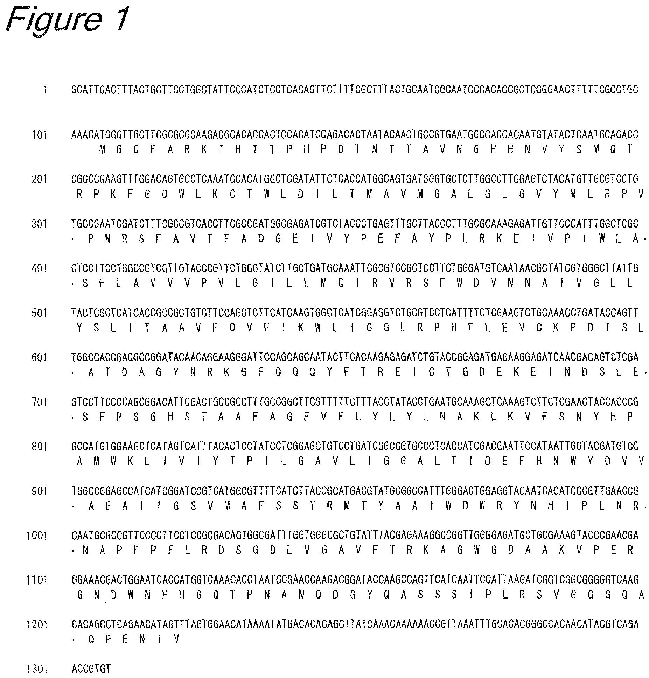

As a result of cDNA screening as described above, the nucleotide sequence of a clone with the longest insert was found to be identical with SEQ ID NO: 1. This plasmid was designated as pB-PAP1. SEQ ID NO: 1 contains a CDS of 1119 bp (SEQ ID NO: 3), thus suggesting that a sequence encoding the full length of PAP homolog was obtained (Figure 1). This gene was designated as the MaPAP1 gene. The deduced amino acid sequence of a protein (MaPAP1p) encoded by this gene is shown in SEQ ID NO: 2.

### (2) Sequence analysis

The nucleotide sequence of the MaPAP1 gene and the deduced amino acid sequence encoded thereby were subjected to homology searches with BLAST and clustalW against known nucleic acid and amino acid sequences. Among sequences hit in the homology search with BLASTX against amino acid sequences registered in GENBANK, a sequence having the lowest E-Value was a putative protein of *Neurospora crassa*-derived DPP1 homolog (Accession No. CAD70721). The amino acid sequence identity was 59.3%, and the identity with a nucleic acid sequence encoding this protein was 57.4%. Likewise, hits were also observed with fungal, plant and animal Mg²⁺-independent phosphatidic acid phosphatase 2 (PAP2) family proteins. PAP2 family enzymes are membrane proteins and are enzymes of the six-transmembrane type. Figure 2 shows an alignment of the amino acid sequences of these PAP2 family proteins with the deduced amino acid sequence encoded by the MaPAP1 gene obtained in this study. PAP2 family enzymes contain three consensus regions, and amino acids essential for their activity are also known. As shown in Figure 2, MaPAP1p was found to also conserve these consensus regions (double-underlined in Figure 2) and the residues essential for activity, i.e., arginine in domain 1 and histidines in domains 2 and 3 (indicated with * in Figure 2).

### Example 3

### Construction of yeast expression vector

To express MaPAP1 in yeast cells, a yeast expression vector was constructed as follows. First, pB-PAP1 was used as a template to perform PCR with ExTaq (Takara Bio Inc., Japan) using primer D-1 (SEQ ID NO: 5) and primer D-3:
CCACTAAACTATGTTCTCAGGC (SEQ ID NO: 7). The resulting DNA fragment of 1.1 kbp was TA-cloned with a TOPO-TA cloning Kit (Invitrogen) and confirmed for its nucleotide sequence. A plasmid carrying the correct CDS nucleotide sequence (SEQ ID NO: 3) of MaPAP1 was designated as pCR-PAP1. The plasmid pCR-PAP1 was partially digested with a restriction enzyme EcoRI. A fragment of approximately 1.1 kbp was excised by agarose gel electrophoresis, purified with a GFX DNA purification Kit(GE Healthcare Bio-Sciences) and ligated to the EcoRI site of yeast expression vector pYE22m (Biosci. Biotech. Biochem., 59, 1221-1228, 1995). The orientation of the inserted DNA fragment was confirmed, and a construct carrying the insert in such an orientation as to cause ORF transcription from the GAPDH promoter of pYE22m was designated as pYE-MAPAP1.

### Example 4

### Yeast transformation

The plasmid pYE22m or pYE-MAPAP1 was used to transform yeast *Saccharomyces cerevisiae* strain EH13-15 (trp1, MATα) (Appl. Microbiol. Biotechnol., 30, 515-520, 1989) by the lithium acetate method. The transformed strains were screened by the ability to grow on SC-Trp agar medium (2% agar) containing, per liter, 6.7 g Yeast nitrogen base w/o amino acids (DIFCO), 20 g glucose and 1.3 g amino acid powder (a mixture of 1.25 g adenine sulfate, 0.6 g arginine, 3 g aspartic acid, 3 g glutamic acid, 0.6 g histidine, 1.8 g leucine, 0.9 g lysine, 0.6 g methionine, 1.5 g phenylalanine, 11.25 g serine, 0.9 g tyrosine, 4.5 g valine, 6 g threonine and 0.6 g uracil).

### Example 5

### Yeast culture

Among the transformed strains obtained with each vector, any two strains (strains c-1 and c-2, or strains MaPAP1-1 and MaPAP1-2) were selected and cultured under the following conditions.

Namely, in the pre-culture step, a loopful of each yeast strain was inoculated from the plate into SC-Trp medium (10 ml) and cultured with shaking at 30°C for 2 days. In the main culture step, the pre-cultured solution (500 µl) was added to 10 ml YPD5 (2% yeast extract, 1% polypeptone, 5% glucose) medium and cultured with shaking at 30°C for 2 days.

### Example 6

### Fatty acid analysis of yeast strains

The cultured yeast solutions were each centrifuged to collect the cells. After washing with 10 ml sterilized water, the cells were collected again by centrifugation and lyophilized. To the lyophilized cells, chloroform:methanol (2:1, 4 ml) was added and stirred vigorously, followed by incubation at 70°C for 1 hour. The cells were separated by centrifugation to collect the solvent. To the remaining cells, chloroform:methanol (2:1, 4 ml) was added again, and the same procedure was repeated to collect the solvent. After lipids were dried up with a SpeedVac, 2 ml chloroform was added to dissolve the lipids. A 200 µl aliquot of this sample was treated by the hydrochloric acid/methanol method to derive fatty acids in the cells into corresponding methyl esters, followed by extraction with hexane. After distilling off hexane, the fatty acids were analyzed by gas chromatography.

The results obtained are shown in Table 2.

Table 2 Fatty acid rate of transformed strains (host: EH13-15)

**[Table 2]**

| Sample name | C-1 | C-2 | MaPAP1-1 | MaPAP1-2 |
|---|---|---|---|---|
| 16:0 | 8.46 | 6.46 | 4.11 | 3.75 |
| 16:1 | 43.26 | 43.10 | 36.11 | 36.00 |
| 18:0 | 4.09 | 4.57 | 5.03 | 4.82 |
| 18:1 | 44.19 | 45.87 | 54.76 | 55.43 |
| 16:1 / 16:0 | 5.11 | 6.67 | 8.79 | 9.60 |
| 18:1 / 16:0 | 5.22 | 7.10 | 13.32 | 14.78 |
| (18:0+18:1) / 16:0 | 5.71 | 7.81 | 14.55 | 16.07 |
| (18:0+18:1) / (16:0+16:1) | 0.93 | 1.02 | 1.49 | 1.52 |

The yeast strains transformed with MaPAP1 (MaPAP1-1 and MaPAP1-2) and the control yeast strains (C-1 and C-2) were compared for their fatty acid rate. In the MaPAP1-transformed yeast, the percentage of oleic acid increased by 20% or more when compared to the control strains. The percentages of palmitic acid and palmitoleic acid both decreased when compared to the control strains, whereas the ratio of the palmitoleic acid content to the palmitic acid content increased slightly when compared to the control strains. Moreover, in the MaPAP1-transformed yeast, the ratio of the oleic acid content to the palmitic acid content and the ratio of the total content of stearic acid and oleic acid to the palmitic acid content both increased when compared to the control strains. Further, in the MaPAP1-transformed yeast, the ratio of C₁₈ fatty acids to C₁₆ fatty acids increased, indicating that fatty acids with longer chain lengths were produced.

Namely, these results indicated that expression of the MaPAP1 gene allowed fatty acid rate alteration.

### Example 7

### Expression analysis in arachidonic acid-producing yeast strains

### (1) Breeding of arachidonic acid-producing yeast strains

To breed arachidonic acid-producing yeast (*Saccharomyces cerevisiae*) strains, the following plasmids were constructed.

First, cDNA prepared from *M. alpina* strain 1S-4 was used as a template to perform PCR with ExTaq using a primer set of Δ12-f and Δ12-r, Δ6-f and Δ6-r, GLELO-f and GLELO-r, or Δ5-f and Δ5-r to thereby amplify the Δ12 fatty acid desaturase gene, the Δ6 fatty acid desaturase gene, the GLELO fatty acid elongase gene or the Δ5 fatty acid desaturase gene in the *M. alpina* strain 1 S-4.
Δ12-f: TCTAGAATGGCACCTCCCAACACTATTG (SEQ ID NO: 8)
Δ12-r: AAGCTTTTACTTCTTGAAAAAGACCACGTC (SEQ ID NO: 9)
Δ6-f: TCTAGAATGGCTGCTGCTCCCAGTGTGAG (SEQ ID NO: 10)
Δ6-r: AAGCTTTTACTGTGCCTTGCCCATCTTGG (SEQ ID NO: 11)
GLELO-f: TCTAGAATGGAGTCGATTGCGCAATTCC (SEQ ID NO: 12)
GLELO-r: GAGCTCTTACTGCAACTTCCTTGCCTTCTC (SEQ ID NO: 13)
Δ5-f: TCTAGAATGGGTGCGGACACAGGAAAAACC (SEQ ID NO: 14)
Δ5-r: AAGCTTTTACTCTTCCTTGGGACGAAGACC (SEQ ID NO: 15)

These genes were cloned with a TOPO-TA-cloning Kit. The clones were confirmed for their nucleotide sequences, and those containing the nucleotide sequences of SEQ ID NOs: 16-19 were designated as plasmids pCR-MAΔ12DS (containing the nucleotide sequence of SEQ ID NO: 16), pCR-MAΔ6DS (containing the nucleotide sequence of SEQ ID NO: 17), pCR-MAGLELO (containing the nucleotide sequence of SEQ ID NO: 18) and pCR- MAΔ5DS (containing the nucleotide sequence of SEQ ID NO: 19), respectively.

On the other hand, a HindIII-digested DNA fragment of approximately 1.2 kb obtained from plasmid pURA34 (JP 2001-120276 A) was inserted into the HindIII site of vector pUC18 which had been digested with restriction enzymes EcoRI and SphI, followed by blunt ending and self-ligation. A clone in which the EcoRI site of the vector was on the 5'-side of URA3 was designated as pUC-URA3. Likewise, a SalI- and XhoI-digested DNA fragment of approximately 2.2 kb obtained from YEp13 was inserted into the SalI site of vector pUC18, and a clone in which the EcoRI site of the vector was on the 5'-side of LUE2 was designated as pUC-LEU2.

Next, the plasmid pCR-MAΔ12DS was digested with a restriction enzyme HindIII and, after blunt ending, was further digested with a restriction enzyme XbaI to obtain a DNA fragment of approximately 1.2 kbp, while vector pESC-URA (STRATAGENE) was digested with a restriction enzyme SacI and, after blunt ending, was further digested with a restriction enzyme SpeI to obtain a DNA fragment of approximately 6.6 kbp. These DNA fragments were ligated to obtain plasmid pESC-U-Δ12. The plasmid pCR-MAΔ6DS was digested with a restriction enzyme XbaI and, after blunt ending, was further digested with a restriction enzyme HindIII to obtain a DNA fragment of approximately 1.6 kbp, while the plasmid pESC-U-Δ12 was digested with a restriction enzyme SalI and, after blunt ending, was further digested with a restriction enzyme HindIII to obtain a DNA fragment of approximately 8 kbp. These DNA fragments were ligated to obtain plasmid pESC-U-Δ12:Δ6. This plasmid was partially digested with a restriction enzyme PvuII, and the resulting fragment of approximately 4.2 kb was inserted into the SmaI site of pUC-URA3 to obtain plasmid pUC-URA-Δ12:Δ6.

Likewise, the plasmid pCR-MAGLELO was digested with restriction enzymes XbaI and SacI to obtain a DNA fragment of approximately 0.95 kbp, while vector pESC-LEU (STRATAGENE) was digested with restriction enzymes XbaI and SacI to obtain a DNA fragment of approximately 7.7 kbp. These DNA fragments were ligated to obtain plasmid pESC-L-GLELO. The plasmid pCR- MAΔ5DS was digested with a restriction enzyme XbaI and, after blunt ending, was further digested with a restriction enzyme HindIII to obtain a DNA fragment of approximately 1.3 kbp, while the plasmid pESC-L-GLELO was digested with a restriction enzyme ApaI and, after blunt ending, was further digested with a restriction enzyme HindIII to obtain a DNA fragment of approximately 8.7 kbp. These DNA fragments were ligated to obtain plasmid pESC-L-GLELO:Δ5. This plasmid was digested with a restriction enzyme PvuII, and the resulting fragment of approximately 3.2 kb was inserted into the SmaI site of pUC-LEU2 to obtain plasmid pUC-LEU-GLELO:Δ5.

*S. cerevisiae* strain YPH499 (STRATAGENE) was co-transformed with plasmid pUC-URA-Δ12:Δ6 and plasmid pUC-LEU-GLELO:Δ5. The transformed strains were screened by the ability to grow on SC-Leu,Ura agar medium (2% agar) containing, per liter, 6.7 g Yeast nitrogen base w/o amino acids (DIFCO), 20 g glucose and 1.3 g amino acid powder (a mixture of 1.25 g adenine sulfate, 0.6 g arginine, 3 g aspartic acid, 3 g glutamic acid, 0.6 g histidine, 0.9 g lysine, 0.6 g methionine, 1.5 g phenylalanine, 11.25 g serine, 0.9 g tyrosine, 4.5 g valine, 6 g threonine and 1.2 g tryptophan). Among the strains thus obtained, any one strain was designated as strain ARA3-1.

### (2) Obtaining and analysis of transformed strains of arachidonic acid-producing yeast

The strain ARA3-1 was transformed respectively with plasmids pYE22m and pYE-MAPAP1. The transformed strains were screened by the ability to grow on SC-Trp,Leu,Ura agar medium (2% agar) containing, per liter, 6.7 g Yeast nitrogen base w/o amino acids (DIFCO), 20 g glucose and 1.3 g amino acid powder (a mixture of 1.25 g adenine sulfate, 0.6 g arginine, 3 g aspartic acid, 3 g glutamic acid, 0.6 g histidine, 0.9 g lysine, 0.6 g methionine, 1.5 g phenylalanine, 11.25 g serine, 0.9 g tyrosine, 4.5 g valine and 6 g threonine). Among the strains thus transformed, any 4 strains were selected for each plasmid.

These strains were each cultured at 30°C for 1 day in the above SC-Trp,Leu,Ura liquid medium (10 ml), 1 ml of which was then cultured at 15°C for 7 days in SG-Trp,Leu,Ura liquid medium (10 ml) containing, per liter, 6.7 g Yeast nitrogen base w/o amino acids (DIFCO), 20 g galactose and 1.3 g amino acid powder (a mixture of 1.25 g adenine sulfate, 0.6 g arginine, 3 g aspartic acid, 3 g glutamic acid, 0.6 g histidine, 0.9 g lysine, 0.6 g methionine, 1.5 g phenylalanine, 11.25 g serine, 0.9 g tyrosine, 4.5 g valine and 6 g threonine), followed by analysis of fatty acids in the cells. Tables 3 and 4 show the fatty acid rate in the cells and the fat and oil content in the cells, respectively.
[Table 3]

**Table 3 Intracellular fatty acid content (%)**

| control | PAP1 |
|---|---|
| 6.32 ± 0.59 | 7.28 ± 1.00 |

[Table 4]

**Table 4 Ratio (%) of γ-linolenic acid, DGLA or ARA to total fatty acid**

| | control | PAP1 |
|---|---|---|
| 18:3(n-6) | 0.54 ± 0.07 | 0.67 ± 0.12 |
| DGLA | 0.33 ± 0.02 | 0.42 ± 0.12 |
| ARA | 0.44 ± 0.03 | 0.53 ± 0.08 |

As shown above, in a case where the *M. alpina-*derived PAP1 gene was highly expressed in arachidonic acid-producing yeast cells, the intracellular fatty acid content was increased when compared to the control strains. Likewise, the ratios of γ-linolenic acid, DGLA and arachidonic acid to total fatty acid were also all increased.

### Example 8

### Vector construction for M alpina expression

The vectors used for *M. alpina* expression were pDuraSC which allows expression of a desired gene from the GAPDH promoter, and pDuraMCS which allows expression of a desired gene from the histone promoter.

To express PAP1 in *M. alpina* cells, vectors were constructed as follows. The plasmid pCR-PAP1 was digested with a restriction enzyme PstI and then partially digested with a restriction enzyme XhoI. Among the resulting DNA fragments, a fragment of approximately 1.1 kb was excised and inserted between the PstI and XhoI site in the multicloning site of vector pDuraSC or pDura5MCS. The resulting contracts were designated as plasmids pDuraSC-PAP1 and pDuraSMCS-PAP1, respectively.

### Obtaining of transformed M alpina strains

Uracil-auxotrophic strain Δura-3 derived from *M. alpina* as described in a patent document (WO2005/019437 entitled "Method of Breeding Lipid-Producing Fungus") was used as a host and transformed with these plasmids by the particle delivery method. For screening of the transformed strains, SC agar medium was used (0.5% Yeast Nitrogen Base w/o Amino Acids and Ammonium Sulfate (Difco), 0.17% ammonium sulfate, 2% glucose, 0.002% adenine, 0.003% tyrosine, 0.0001% methionine, 0.0002% arginine, 0.0002% histidine, 0.0004% lysine, 0.0004% tryptophan, 0.0005% threonine, 0.0006% isoleucine, 0.0006% leucine, 0.0006% phenylalanine, and 2% agar).

### Evaluation of M. alpina transformants

The resulting transformed strains were each inoculated into 4 ml GY medium (2% glucose, 1% yeast extract) and cultured with shaking at 28°C for 3 or 4 days. The cells were collected by filtration, and RNA was extracted with an RNeasy plant kit (QIAGEN). A SuperScript First-Strand system for RT-PCR (Invitrogen) was used to synthesize cDNA. To confirm expression from the introduced construct and total expression for each gene, RT-PCR was performed with the following primer sets.

### Strains transformed with plasmid pDuraSC-PAP1

Primers used for confirmation of PAP1 expression from the introduced construct:
MaGAPDHpfw: CACACCACACATTCAACATC (SEQ ID NO: 20); and
D2: CGAAGCCGGCAAAGGCGGCAGTCG (SEQ ID NO: 21)

Primers used for confirmation of total PAP1 expression:
D1: CATGGGTTGCTTCGCGCGCAAGACG (SEQ ID NO: 22); and
D2

### Strains transformed with plasmid pDuraSMCS-PAP1

Primers used for confirmation of PAP 1 expression from the introduced construct:
PD4P: CGCATCCCGCAAACACACAC (SEQ ID NO: 23); and
D2

Primers used for confirmation of total PAP1 expression:
D1; and
D2

Based on the results of the above RT-PCR, transformants showing high level expression of each gene both in expression from the introduced construct and in total expression were selected: strains Gp-PAP1-49 and Hp-PAP1-2 from those transformed with plasmids pDuraSC-PAP1 and pDura5MCS-PAP1, respectively.

These strains were each inoculated into GY medium (4 ml) and cultured with shaking at 28°C at 125 rpm. On day 4 of culture, all cells were collected by filtration and lyophilized. A portion (about 10-20 mg) of the dried cells was treated by the hydrochloric acid/methanol method to derive fatty acids in the cells into corresponding methyl esters, followed by extraction with hexane. After distilling off hexane, the fatty acids were analyzed by gas chromatography. The intracellular fatty acid content and the arachidonic acid production per medium are summarized in Tables 5-7 below.

**[Table 5]**

| Table 5 Intracellular fatty acid content (%) | | |
|---|---|---|
| Hp-PAP1-2 | Gp-PAP1-49 | 1S-4 |
| 25.23 ± 1.84 | 26.64 ± 2.56 | 22.09 ± 3.20 |

**[Table 6]**

| Table 6 Ratio (%) of arachidonic acid to total fatty acid | | |
|---|---|---|
| Hp-PAP1-2 | Gp-PAP1-49 | 1S-4 |
| 36.03 ± 1.38 | 38.06 ± 1.09 | 35.52 ± 1.47 |

**[Table 7]**

| Table 7 Arachidonic acid production (g/L) per medium | | |
|---|---|---|
| Hp-PAP1-2 | Gp-PAP1-49 | 1 S-4 |
| 1.05±0.14 | 1.15 ± 0.13 | 0.88 ± 0.17 |

As shown above, high expression of the PAP1 gene in *M. alpina* allowed an increase in both the intracellular fatty acid content and the ratio of arachidonic acid to total fatty acid. Moreover, it was also possible to increase arachidonic acid production per medium.

### SEQUENCE LISTING FREE TEXT

SEQ ID NO: 5: primer
SEQ ID NO: 6: primer
SEQ ID NO: 7: primer
SEQ ID NO: 8: primer
SEQ ID NO: 9: primer
SEQ ID NO: 10: primer
SEQ ID NO: 11: primer
SEQ ID NO: 12: primer
SEQ ID NO: 13: primer
SEQ ID NO: 14: primer
SEQ ID NO: 15: primer

SEQ ID NO: 20: primer
SEQ ID NO: 21: primer
SEQ ID NO: 22: primer
SEQ ID NO: 23: primer

## Claims

1. A protein shown in any one of (a) to (e) below:
(a) a protein consisting of the amino acid sequence shown in SEQ ID NO: 2;
(b) a protein which consists of an amino acid sequence with deletion, substitution or addition of 1 to 50 amino acids in SEQ ID NO: 2 and which has phosphatidic acid phosphatase activity;
(c) a protein which consists of an amino acid sequence sharing an identity of 85% or more with the amino acid sequence consisting of SEQ ID NO: 2 and which has phosphatidic acid phosphatase activity;
(d) a protein which consists of an amino acid sequence with deletion, substitution or addition of 1 to 50 amino acids in SEQ ID NO: 2 and which has the ability to yield a fatty acid rate ensuring a higher ratio of at least one or more of (i) to (vi) shown below in the fatty acid rate of a host expressing a protein consisting of the amino acid sequence than in the fatty acid rate of a host not expressing the protein:
(i) the oleic acid content;
(ii) the ratio of the palmitoleic acid content to the palmitic acid content;
(iii) the ratio of the oleic acid content to the palmitic acid content;
(iv) the ratio of the total content of stearic acid and oleic acid to the palmitic acid content;
(v) the ratio of the C₁₈ fatty acid content to the C₁₆ fatty acid content; and
(vi) the ratio of the arachidonic acid, dihomo-γ-linolenic acid and/or γ-linolenic acid content; or
(e) a protein which consists of an amino acid sequence sharing an identity of 85% or more with the amino acid sequence consisting of SEQ ID NO: 2 and which has the ability to yield a fatty acid rate ensuring a higher ratio of at least one or more of (i) to (vi) shown below in the fatty acid rate of a host expressing a protein consisting of the amino acid sequence than in the fatty acid rate of a host not expressing the protein:
(i) the oleic acid content;
(ii) the ratio of the palmitoleic acid content to the palmitic acid content;
(iii) the ratio of the oleic acid content to the palmitic acid content;
(iv) the ratio of the total content of stearic acid and oleic acid to the palmitic acid content;
(v) the ratio of the C₁₈ fatty acid content to the C₁₆ fatty acid content; and
(vi) the ratio of the arachidonic acid, dihomo-γ-linolenic acid and/or γ-linolenic acid content.

2. A nucleic acid comprising a nucleotide sequence shown in any one of (a) to (i) below:
(a) the nucleotide sequence shown in SEQ ID NO: 4;
(b) the nucleotide sequence shown in SEQ ID NO: 1;
(c) a nucleotide sequence which encodes a protein according to claim 1(a);
(d) a nucleotide sequence which encodes a protein according to claim 1(b);
(e) a nucleotide sequence which encodes a protein according to claim 1(c);
(f) a nucleotide sequence which encodes a protein according to claim 1(d);
(g) a nucleotide sequence which encodes a protein according to claim 1(e);
(h) a nucleotide sequence which consists of a nucleotide sequence sharing an identity of 85% or more with the nucleotide sequence consisting of SEQ ID NO: 4 and which encodes a protein having phosphatidic acid phosphatase activity; or
(i) a nucleotide sequence which consists of a nucleotide sequence sharing an identity of 85% or more with the nucleotide sequence consisting of SEQ ID NO: 4 and which encodes the following protein:
a protein which has the ability to yield a fatty acid rate ensuring a higher ratio of at least one or more of (i) to (vi) shown below in the fatty acid rate of a host expressing the protein than in the fatty acid rate of a host not expressing the protein:
(i) the oleic acid content;
(ii) the ratio of the palmitoleic acid content to the palmitic acid content;
(iii) the ratio of the oleic acid content to the palmitic acid content;
(iv) the ratio of the total content of stearic acid and oleic acid to the palmitic acid content;
(v) the ratio of the C₁₈ fatty acid content to the C₁₆ fatty acid content; and
(vi) the ratio of the arachidonic acid, dihomo-γ-linolenic acid and/or γ-linolenic acid content.

3. A nucleic acid according to claim 2 comprising a nucleotide sequence shown in any one of (a) to (d) below:
(a) the nucleotide sequence according to claim 2(d), wherein the number of amino acids to be deleted, substituted or added in the protein according to claim 1(b) is 1 to 10;
(b) the nucleotide sequence according to claim 2(e), wherein the amino acid sequence of the protein according to claim 1(c) shares an identity of 90% or more with the amino acid sequence consisting of SEQ ID NO: 2;
(c) the nucleotide sequence according to claim 2(f), wherein the number of amino acids to be deleted, substituted or added in the protein according to claim 1(d) is 1 to 10; or
(d) the nucleotide sequence according to claim 2(g), wherein the amino acid sequence of the protein according to claim 1(e) shares an identity of 90% or more with the amino acid sequence consisting of SEQ ID NO: 2.

4. A recombinant vector comprising the nucleic acid according to claim 2 or 3.

5. A transformant transformed with the recombinant vector according to claim 4.

6. A method for preparing a fatty acid composition comprising culturing the transformant according to claim 5 and collecting the fatty acid composition contained in the cultured product obtained by culturing the transformant according to claim 5.

## Patentansprüche

1. Protein wie in einem von (a) bis (e) nachfolgend gezeigt:
(a) ein Protein, das aus der in SEQ ID NO:2 gezeigten Aminosäuresequenz besteht;
(b) ein Protein, das aus einer Aminosäuresequenz mit Deletion, Substitution oder Hinzufügung von 1 bis 50 Aminosäuren in SEQ ID NO:2 besteht und Phosphatidsäure-Phosphatase-Aktivität aufweist;
(c) ein Protein, das aus einer Aminosäuresequenz besteht, die mit der Aminosäuresequenz, die aus SEQ ID NO:2 besteht, 85% oder höhere Identität hat und die Phosphatidsäure-Phosphatase-Aktivität aufweist;
(d) ein Protein, das aus einer Aminosäuresequenz mit Deletion, Substitution oder Hinzufügung von 1 bis 50 Aminosäuren in SEQ ID NO:2 besteht und die Fähigkeit hat, einen Fettsäureanteil zu liefern, der ein höheres Verhältnis gemäß mindestens einem von (i) bis (vi) wie nachfolgend gezeigt im Fettsäureanteil eines Wirts gewährleistet, der ein Protein exprimiert, das aus dieser Aminosäuresequenz besteht, als in dem Fettsäureanteil eines Wirts, der das Protein nicht exprimiert:
(i) Oleinsäuregehalt;
(ii) Verhältnis des Palmitoleinsäuregehalts zum Palmitinsäuregehalt;
(iii) Verhältnis des Oleinsäuregehalts zum Palmitinsäuregehalt;
(iv) Verhältnis des Gesamtgehalts an Stearinsäure und Oleinsäure zum Palmitinsäuregehalt;
(v) Verhältnis des C₁₈-Fettsäuregehalts zum C₁₆-Fettsäuregehalt; und
(vi) Verhältnis des Arachidonsäure-, Dihomo-γ-Linolensäure- und/oder γ-Linolensäuregehalts; oder
(e) ein Protein, das aus einer Aminosäuresequenz besteht, die 85% oder höhere Identität mit der Aminosäuresequenz hat, die aus SEQ ID NO:2 besteht und die die Fähigkeit hat, einen Fettsäureanteil zu liefern, der ein höheres Verhältnis gemäß mindestens einem von (i) bis (vi) wie nachfolgend gezeigt im Fettsäureanteil eines Wirts gewährleistet, der ein Protein exprimiert, das aus dieser Aminosäuresequenz besteht, als in dem Fettsäureanteil eines Wirts, der das Protein nicht exprimiert:
(i) Oleinsäuregehalt;
(ii) Verhältnis des Palmitoleinsäuregehalts zum Palmitinsäuregehalt;
(iii) Verhältnis des Oleinsäuregehalts zum Palmitinsäuregehalt;
(iv) Verhältnis des Gesamtgehalts an Stearinsäure und Oleinsäure zum Palmitinsäuregehalt;
(v) Verhältnis des C₁₈-Fettsäuregehalts zum C₁₆-Fettsäuregehalt; und
(vi) Verhältnis des Arachidonsäure-, Dihomo-γ-Linolensäure- und/oder γ-Linolensäuregehalts.

2. Nucleinsäure, die eine wie in einem von (a) bis (i) nachfolgend gezeigte Nucleotidsequenz umfasst:
(a) die in SEQ ID NO:4 gezeigte Nucleotidsequenz;
(b) die in SEQ ID NO:1 gezeigte Nucleotidsequenz;
(c) eine Nucleotidsequenz, die ein Protein nach Anspruch 1(a) codiert;
(d) eine Nucleotidsequenz, die ein Protein nach Anspruch 1(b) codiert;
(e) eine Nucleotidsequenz, die ein Protein nach Anspruch 1(c) codiert;
(f) eine Nucleotidsequenz, die ein Protein nach Anspruch 1(d) codiert;
(g) eine Nucleotidsequenz, die ein Protein nach Anspruch 1(e) codiert;
(h) eine Nucleotidsequenz, die aus einer Nucleotidsequenz besteht, die 85% oder höhere Identität mit der Nucleotidsequenz hat, die aus der SEQ ID NO:4 besteht und die ein Protein codiert, das Phosphatidsäure-Phosphatase-Aktivität aufweist; oder
(i) eine Nucleotidsequenz, die aus einer Nucleotidsequenz besteht, die 85% oder höhere Identität mit der Nucleotidsequenz hat, die aus der SEQ ID NO:4 besteht und die das folgende Protein codiert:
ein Protein, das die Fähigkeit hat, einen Fettsäureanteil zu liefern, der ein höheres Verhältnis gemäß mindestens einem von (i) bis (vi) wie nachfolgend gezeigt im Fettsäureanteil eines Wirts gewährleistet, der das Protein exprimiert, als in dem Fettsäureanteil eines Wirts, der das Protein nicht exprimiert:
(i) Oleinsäuregehalt;
(ii) Verhältnis des Palmitoleinsäuregehalts zum Palmitinsäuregehalt;
(iii) Verhältnis des Oleinsäuregehalts zum Palmitinsäuregehalt;
(iv) Verhältnis des Gesamtgehalts an Stearinsäure und Oleinsäure zum Palmitinsäuregehalt;
(v) Verhältnis des C₁₈-Fettsäuregehalts zum C₁₆-Fettsäuregehalt; und
(vi) Verhältnis des Arachidonsäure-, Dihomo-γ-Linolensäure- und/oder γ-Linolensäuregehalts.

3. Nucleinsäure nach Anspruch 2, die eine wie in einem von (a) bis (d) nachfolgend gezeigte Nucleotidsequenz umfasst:
(a) die Nucleotidsequenz nach Anspruch 2(d), wobei die Anzahl der Aminosäuren, die in dem Protein nach Anspruch 1(b) zu deletieren, substituieren oder hinzuzufügen sind, 1 bis 10 ist;
(b) die Nucleotidsequenz nach Anspruch 2(e), wobei die Aminosäuresequenz des Proteins nach Anspruch 1(c) 90% oder höhere Identität mit der Aminosäuresequenz hat, die aus SEQ ID NO:2 besteht;
(c) die Nucleotidsequenz nach Anspruch 2(f), wobei die Anzahl der Aminosäuren, die in dem Protein nach Anspruch 1(d) zu deletieren, substituieren oder hinzuzufügen sind, 1 bis 10 ist; oder
(d) die Nucleotidsequenz nach Anspruch 2(g), wobei die Aminosäuresequenz des Proteins nach Anspruch 1(e) 90% oder höhere Identität mit der Aminosäuresequenz hat, die aus SEQ ID NO:2 besteht.

4. Rekombinanter Vektor, der die Nucleinsäure nach Anspruch 2 oder 3 umfasst.

5. Transformante, die mit dem rekombinanten Vektor nach Anspruch 4 transformiert ist.

6. Verfahren zur Herstellung einer Fettsäurezusammensetzung, umfassend das Züchten der Transformante nach Anspruch 5 und Gewinnen der Fettsäurezusammensetzung, die in dem kultivierten Produkt enthalten ist, das durch Züchten der Transformante nach Anspruch 5 erhalten wird.

## Revendications

1. Protéine montrée dans l'une quelconque de (a) à (e) ci-dessous :
(a) une protéine consistant en la séquence d'acides aminés montrée dans SEQ ID NO: 2;
(b) une protéine qui consiste en une séquence d'acides aminés comportant une délétion, une substitution ou une addition de 1 à 50 acides aminés dans SEQ ID NO: 2 et qui possède une activité acide phosphatidique phosphatase ;
(c) une protéine qui consiste en une séquence d'acides aminés partageant une identité de 85 % ou plus avec la séquence d'acides aminés consistant en SEQ ID NO: 2 et qui possède une activité acide phosphatidique phosphatase ;
(d) une protéine qui consiste en une séquence d'acides aminés comportant une délétion, une substitution ou une addition de 1 à 50 acides aminés dans SEQ ID NO: 2 et qui possède une aptitude à produire un taux d'acide gras garantissant un rapport plus élevé d'au moins un ou plusieurs de (i) à (vi) montré ci-dessous dans le taux d'acide gras d'un hôte exprimant une protéine consistant en la séquence d'acides aminés par rapport au taux d'acide gras d'un hôte n'exprimant pas la protéine :
(i) la teneur en acide oléique ;
(ii) le rapport de la teneur en acide palmitoléique sur la teneur en acide palmitique ;
(iii) le rapport de la teneur en acide oléique sur la teneur en acide palmitique ;
(iv) le rapport de la teneur totale en acide stéarique et en acide oléique sur la teneur en acide palmitique ;
(v) le rapport de la teneur en acide gras C₁₈ sur la teneur en acide gras C₁₆ ; et
(vi) le rapport de la teneur en acide arachidonique, acide dihomo-γ-linolénique et/ou acide γ-linolénique ; ou
(e) une protéine qui consiste en une séquence d'acides aminés partageant une identité de 85 % ou plus avec la séquence d'acides aminés consistant en SEQ ID NO: 2 et qui possède une aptitude à produire un taux d'acide gras garantissant un rapport plus élevé d'au moins un ou plusieurs de (i) à (vi) montré ci-dessous dans le taux d'acide gras d'un hôte exprimant une protéine consistant en la séquence d'acides aminés par rapport au taux d'acide gras d'un hôte n'exprimant pas la protéine :
(i) la teneur en acide oléique ;
(ii) le rapport de la teneur en acide palmitoléique sur la teneur en acide palmitique ;
(iii) le rapport de la teneur en acide oléique sur la teneur en acide palmitique ;
(iv) le rapport de la teneur totale en acide stéarique et en acide oléique sur la teneur en acide palmitique ;
(v) le rapport de la teneur en acide gras C₁₈ sur la teneur en acide gras C₁₆ ; et
(vi) le rapport de la teneur en acide arachidonique, acide dihomo-γ-linolénique et/ou acide γ-linolénique.

2. Acide nucléique comprenant une séquence de nucléotides montrée dans l'une quelconque de (a) à (i) ci-dessous :
(a) la séquence de nucléotides montrée dans SEQ ID NO: 4 ;
(b) la séquence de nucléotides montrée dans SEQ ID NO: 1 ;
(c) une séquence de nucléotides qui code pour une protéine selon la revendication 1(a) ;
(d) une séquence de nucléotides qui code pour une protéine selon la revendication 1(b) ;
(e) une séquence de nucléotides qui code pour une protéine selon la revendication 1(c);
(f) une séquence de nucléotides qui code pour une protéine selon la revendication 1(d);
(g) une séquence de nucléotides qui code pour une protéine selon la revendication 1(e) ;
(h) une séquence de nucléotides qui consiste en une séquence de nucléotides partageant une identité de 85 % ou plus avec la séquence de nucléotides consistant en SEQ ID NO: 4 et qui code pour une protéine possédant une activité acide phosphatidique phosphatase ; ou
(i) une séquence de nucléotides qui consiste en une séquence de nucléotides partageant une identité de 85 % ou plus avec la séquence de nucléotides consistant en SEQ ID NO: 4 et qui code pour la protéine suivante :
une protéine qui possède une aptitude à produire un taux d'acide gras garantissant un rapport plus élevé d'au moins un ou plusieurs de (i) à (vi) montré ci-dessous dans le taux d'acide gras d'un hôte exprimant la protéine par rapport au taux d'acide gras d'un hôte n'exprimant pas la protéine :
(i) la teneur en acide oléique ;
(ii) le rapport de la teneur en acide palmitoléique sur la teneur en acide palmitique ;
(iii) le rapport de la teneur en acide oléique sur la teneur en acide palmitique ;
(iv) le rapport de la teneur totale en acide stéarique et en acide oléique sur la teneur en acide palmitique ;
(v) le rapport de la teneur en acide gras C₁₈ sur la teneur en acide gras C₁₆ ; et
(vi) le rapport de la teneur en acide arachidonique, acide dihomo-γ-linolénique et/ou acide γ-linolénique.

3. Acide nucléique selon la revendication 2 comprenant une séquence de nucléotides montrée dans l'une quelconque de (a) à (d) ci-dessous :
(a) la séquence de nucléotides selon la revendication 2(d), dans laquelle le nombre d'acides aminés devant être supprimés, substitués ou ajoutés dans la protéine selon la revendication 1(b) est 1 à 10 ;
(b) la séquence de nucléotides selon la revendication 2(e), dans laquelle la séquence d'acides aminés de la protéine selon la revendication 1(c) partage une identité de 90 % ou plus avec la séquence d'acides aminés consistant en SEQ ID NO: 2 ;
(c) la séquence de nucléotides selon la revendication 2(f), dans laquelle le nombre d'acides aminés devant être supprimés, substitués ou ajoutés dans la protéine selon la revendication 1(d) est 1 à 10 ; ou
(d) la séquence de nucléotides selon la revendication 2(g), dans laquelle la séquence d'acides aminés de la protéine selon la revendication 1(e) partage une identité de 90 % ou plus avec la séquence d'acides aminés consistant en SEQ ID NO: 2.

4. Vecteur recombinant comprenant l'acide nucléique selon la revendication 2 ou 3.

5. Transformant transformé avec le vecteur recombinant selon la revendication 4.

6. Procédé pour préparer une composition d'acide gras comprenant la mise en culture du transformant selon la revendication 5 et la collecte de la composition d'acide gras contenue dans le produit mis en culture obtenu par la mise en culture du transformant selon la revendication 5.


### SEQUENCE LISTING

<110> Suntory Limited
<120> A phosphatidic acid phosphatase (PAP) homolog and use thereof
<130> YCT-1378
<150> JP 2007-181899
   <151> 2007-07-11
<160> 23
<170> PatentIn version 3.1
<210> 1
   <211> 1307
   <212> DNA
   <213> Mortierella alpina
<220>
   <221> CDS
   <222> (105)..(1223)
   <223>
<400> 1
   gcattcactt tactgcttcc tggctattcc catctcctca cagttctttt cgctttactg     60
   caatcgcaat cccacaccgc tcgggaactt tttcgcctgc aaac atg ggt tgc ttc     116
                                                    Met Gly Cys Phe
                                                    1
   gcg cgc aag acg cac acc act cca cat cca gac act aat aca act gcc       164
   Ala Arg Lys Thr His Thr Thr Pro His Pro Asp Thr Asn Thr Thr Ala
   5                   10                  15                  20
   gtg aat ggc cac cac aat gta tac tca atg cag acc cgg ccg aag ttt       212
   Val Asn Gly His His Asn Val Tyr Ser Met Gln Thr Arg Pro Lys Phe
                   25                  30                  35
   gga cag tgg ctc aaa tgc aca tgg ctc gat att ctc acc atg gca gtg       260
   Gly Gln Trp Leu Lys Cys Thr Trp Leu Asp Ile Leu Thr Met Ala Val
               40                  45                  50
   atg ggt gct ctt ggc ctt gga gtc tac atg ttg cgt cct gtg ccg aat       308
   Met Gly Ala Leu Gly Leu Gly Val Tyr Met Leu Arg Pro Val Pro Asn
           55                  60                  65
   cga tct ttc gcc gtc acc ttc gcc gat ggc gag atc gtc tac cct gag       356
   Arg Ser Phe Ala Val Thr Phe Ala Asp Gly Glu Ile Val Tyr Pro Glu
       70                  75                  80
   ttt gct tac cct ttg cgc aaa gag att gtt ccc att tgg ctc gcc tcc       404
   Phe Ala Tyr Pro Leu Arg Lys Glu Ile Val Pro Ile Trp Leu Ala Ser
   85                  90                  95                  100
   ttc ctg gcc gtc gtt gta ccc gtt ctg ggt atc ttg ctg atg caa att       452
   Phe Leu Ala Val Val Val Pro Val Leu Gly Ile Leu Leu Met Gln Ile
                   105                 110                 115
   cgc gtc cgc tcc ttc tgg gat gtc aat aac gct atc gtg ggc tta ttg       500
   Arg Val Arg Ser Phe Trp Asp Val Asn Asn Ala Ile Val Gly Leu Leu
               120                 125                 130
   tac tcg ctc atc acc gcc gct gtc ttc cag gtc ttc atc aag tgg ctc      548
   Tyr Ser Leu Ile Thr Ala Ala Val Phe Gln Val Phe Ile Lys Trp Leu
           135                 140                 145
   atc gga ggt ctg cgt cct cat ttt ctc gaa gtc tgc aaa cct gat acc      596
   Ile Gly Gly Leu Arg Pro His Phe Leu Glu Val Cys Lys Pro Asp Thr
       150                 155                 160
   agt ttg gcc acc gac gcc gga tac aac agg aag gga ttc cag cag caa      644
   Ser Leu Ala Thr Asp Ala Gly Tyr Asn Arg Lys Gly Phe Gln Gln Gln
   165                 170                 175                 180
   tac ttc aca aga gag atc tgt acc gga gat gag aag gag atc aac gac      692
   Tyr Phe Thr Arg Glu Ile Cys Thr Gly Asp Glu Lys Glu Ile Asn Asp
                   185                 190                 195
   agt ctc gag tcc ttc ccc agc gga cat tcg act gcc gcc ttt gcc ggc      740
   Ser Leu Glu Ser Phe Pro Ser Gly His Ser Thr Ala Ala Phe Ala Gly
               200                 205                 210
   ttc gkt ttt ctt tac cta tac ctg aat gca aag ctc aaa gtc ttc tcg      788
   Phe Xaa Phe Leu Tyr Leu Tyr Leu Asn Ala Lys Leu Lys Val Phe Ser
           215                 220                 225
   aac tac cac ccg gcc atg tgg aag ctc ata gtc att tac act cct atc      836
   Asn Tyr His Pro Ala Met Trp Lys Leu Ile Val Ile Tyr Thr Pro Ile
       230                 235                 240
   ctc gga gct gtc ctg atc ggc ggt gcc ctc acc atc gac gaa ttc cat      884
   Leu Gly Ala Val Leu Ile Gly Gly Ala Leu Thr Ile Asp Glu Phe His
   245                 250                 255                 260
   aat tgg tac gat gtc gtg gcc gga gcc atc atc gga tcc gtc atg gcg      932
   Asn Trp Tyr Asp Val Val Ala Gly Ala Ile Ile Gly Ser Val Met Ala
                   265                 270                 275
   ttt tca tct tac cgc atg acg tat gcg gcc att tgg gac tgg agg tac      980
   Phe Ser Ser Tyr Arg Met Thr Tyr Ala Ala Ile Trp Asp Trp Arg Tyr
               280                 285                 290
   aat cac atc ccg ttg aac cgc aat gcg ccg ttc ccc ttc ctc cgc gac     1028
   Asn His Ile Pro Leu Asn Arg Asn Ala Pro Phe Pro Phe Leu Arg Asp
           295                 300                 305
   agt ggc gat ttg gtg ggc gct gta ttt acg aga aag gcc ggt tgg gga     1076
   Ser Gly Asp Leu Val Gly Ala Val Phe Thr Arg Lys Ala Gly Trp Gly
       310                 315                 320
   gat gct gcg aaa gta ccc gaa cga gga aac gac tgg aat cac cat ggt     1124
   Asp Ala Ala Lys Val Pro Glu Arg Gly Asn Asp Trp Asn His His Gly
   325                 330                 335                 340
   caa aca cct aat gcg aac caa gac gga tac caa gcc agt tca tca att     1172
   Gln Thr Pro Asn Ala Asn Gln Asp Gly Tyr Gln Ala Ser Ser Ser Ile
                   345                 350                 355
   cca tta aga tcg gtc ggc ggg ggt caa gca cag cct gag aac ata gtt     1220
   Pro Leu Arg Ser Val Gly Gly Gly Gln Ala Gln Pro Glu Asn Ile Val
   360 365 370
   tag tggaacataa aatatgacac acagcttatc aaacaaaaaa ccgttaaatt 1273
   tgcacacggg ccacaacata cgtcagaacc gtgt 1307
<210> 2
   <211> 372
   <212> PRT
   <213> Mortierella alpina
<220>
   <221> misc_feature
   <222> (214)..(214)
   <223> The 'Xaa' at location 214 stands for Gly, or Val.
<400> 2
   Met Gly Cys Phe Ala Arg Lys Thr His Thr Thr Pro His Pro Asp Thr
   1               5                   10                  15
   Asn Thr Thr Ala Val Asn Gly His His Asn Val Tyr Ser Met Gln Thr
               20                  25                  30
   Arg Pro Lys Phe Gly Gln Trp Leu Lys Cys Thr Trp Leu Asp Ile Leu
           35                  40                  45
   Thr Met Ala Val Met Gly Ala Leu Gly Leu Gly Val Tyr Met Leu Arg
       50                  55                  60
   Pro Val Pro Asn Arg Ser Phe Ala Val Thr Phe Ala Asp Gly Glu Ile
   65                  70                  75                  80
   Val Tyr Pro Glu Phe Ala Tyr Pro Leu Arg Lys Glu Ile Val Pro Ile
                   85                  90                  95
   Trp Leu Ala Ser Phe Leu Ala Val Val Val Pro Val Leu Gly Ile Leu
               100                 105                 110
   Leu Met Gln Ile Arg Val Arg Ser Phe Trp Asp Val Asn Asn Ala Ile
           115                 120                 125
   Val Gly Leu Leu Tyr Ser Leu Ile Thr Ala Ala Val Phe Gln Val Phe
       130                 135                 140
   Ile Lys Trp Leu Ile Gly Gly Leu Arg Pro His Phe Leu Glu Val Cys
   145                 150                 155                 160
   Lys Pro Asp Thr Ser Leu Ala Thr Asp Ala Gly Tyr Asn Arg Lys Gly
                   165                 170                 175
   Phe Gln Gln Gln Tyr Phe Thr Arg Glu Ile Cys Thr Gly Asp Glu Lys
               180                 185                 190
   Glu Ile Asn Asp Ser Leu Glu Ser Phe Pro Ser Gly His Ser Thr Ala
           195                 200                 205
   Ala Phe Ala Gly Phe Xaa Phe Leu Tyr Leu Tyr Leu Asn Ala Lys Leu
       210                 215                 220
   Lys Val Phe Ser Asn Tyr His Pro Ala Met Trp Lys Leu Ile Val Ile
   225                 230                 235                 240
   Tyr Thr Pro Ile Leu Gly Ala Val Leu Ile Gly Gly Ala Leu Thr Ile
                   245                 250                 255
   Asp Glu Phe His Asn Trp Tyr Asp Val Val Ala Gly Ala Ile Ile Gly
               260                 265                 270
   Ser Val Met Ala Phe Ser Ser Tyr Arg Met Thr Tyr Ala Ala Ile Trp
           275                 280                 285
   Asp Trp Arg Tyr Asn His Ile Pro Leu Asn Arg Asn Ala Pro Phe Pro
       290                 295                 300
   Phe Leu Arg Asp Ser Gly Asp Leu Val Gly Ala Val Phe Thr Arg Lys
   305                 310                 315                 320
   Ala Gly Trp Gly Asp Ala Ala Lys Val Pro Glu Arg Gly Asn Asp Trp
                   325                 330                 335
   Asn His His Gly Gln Thr Pro Asn Ala Asn Gln Asp Gly Tyr Gln Ala
               340                 345                 350
   Ser Ser Ser Ile Pro Leu Arg Ser Val Gly Gly Gly Gln Ala Gln Pro
           355                 360                 365
   Glu Asn Ile Val
       370
<210> 3
   <211> 1119
   <212> DNA
   <213> Mortierella alpina
<400> 3
   atgggttgct tcgcgcgcaa gacgcacacc actccacatc cagacactaa tacaactgcc      60
   gtgaatggcc accacaatgt atactcaatg cagacccggc cgaagtttgg acagtggctc     120
   aaatgcacat ggctcgatat tctcaccatg gcagtgatgg gtgctcttgg ccttggagtc     180
   tacatgttgc gtcctgtgcc gaatcgatct ttcgccgtca ccttcgccga tggcgagatc     240
   gtctaccctg agtttgctta ccctttgcgc aaagagattg ttcccatttg gctcgcctcc     300
   ttcctggccg tcgttgtacc cgttctgggt atcttgctga tgcaaattcg cgtccgctcc     360
   ttctgggatg tcaataacgc tatcgtgggc ttattgtact cgctcatcac cgccgctgtc     420
   ttccaggtct tcatcaagtg gctcatcgga ggtctgcgtc ctcattttct cgaagtctgc     480
   aaacctgata ccagtttggc caccgacgcc ggatacaaca ggaagggatt ccagcagcaa     540
   tacttcacaa gagagatctg taccggagat gagaaggaga tcaacgacag tctcgagtcc     600
   ttccccagcg gacattcgac tgccgccttt gccggcttcg kttttcttta cctatacctg     660
   aatgcaaagc tcaaagtctt ctcgaactac cacccggcca tgtggaagct catagtcatt     720
   tacactccta tcctcggagc tgtcctgatc ggcggtgccc tcaccatcga cgaattccat     780
   aattggtacg atgtcgtggc cggagccatc atcggatccg tcatggcgtt ttcatcttac     840
   cgcatgacgt atgcggccat ttgggactgg aggtacaatc acatcccgtt gaaccgcaat     900
   gcgccgttcc ccttcctccg cgacagtggc gatttggtgg gcgctgtatt tacgagaaag     960
   gccggttggg gagatgctgc gaaagtaccc gaacgaggaa acgactggaa tcaccatggt    1020
   caaacaccta atgcgaacca agacggatac caagccagtt catcaattcc attaagatcg    1080
   gtcggcgggg gtcaagcaca gcctgagaac atagtttag                          1119
<210> 4
   <211> 1116
   <212> DNA
   <213> Mortierella alpina
<400> 4
   atgggttgct tcgcgcgcaa gacgcacacc actccacatc cagacactaa tacaactgcc      60
   gtgaatggcc accacaatgt atactcaatg cagacccggc cgaagtttgg acagtggctc     120
   aaatgcacat ggctcgatat tctcaccatg gcagtgatgg gtgctcttgg ccttggagtc     180
   tacatgttgc gtcctgtgcc gaatcgatct ttcgccgtca ccttcgccga tggcgagatc     240
   gtctaccctg agtttgctta ccctttgcgc aaagagattg ttcccatttg gctcgcctcc     300
   ttcctggccg tcgttgtacc cgttctgggt atcttgctga tgcaaattcg cgtccgctcc     360
   ttctgggatg tcaataacgc tatcgtgggc ttattgtact cgctcatcac cgccgctgtc     420
   ttccaggtct tcatcaagtg gctcatcgga ggtctgcgtc ctcattttct cgaagtctgc     480
   aaacctgata ccagtttggc caccgacgcc ggatacaaca ggaagggatt ccagcagcaa     540
   tacttcacaa gagagatctg taccggagat gagaaggaga tcaacgacag tctcgagtcc     600
   ttccccagcg gacattcgac tgccgccttt gccggcttcg kttttcttta cctatacctg     660
   aatgcaaagc tcaaagtctt ctcgaactac cacccggcca tgtggaagct catagtcatt     720
   tacactccta tcctcggagc tgtcctgatc ggcggtgccc tcaccatcga cgaattccat     780
   aattggtacg atgtcgtggc cggagccatc atcggatccg tcatggcgtt ttcatcttac     840
   cgcatgacgt atgcggccat ttgggactgg aggtacaatc acatcccgtt gaaccgcaat     900
   gcgccgttcc ccttcctccg cgacagtggc gatttggtgg gcgctgtatt tacgagaaag     960
   gccggttggg gagatgctgc gaaagtaccc gaacgaggaa acgactggaa tcaccatggt    1020
   caaacaccta atgcgaacca agacggatac caagccagtt catcaattcc attaagatcg    1080
   gtcggcgggg gtcaagcaca gcctgagaac atagtt                              1116
<210> 5
   <211> 25
   <212> DNA
   <213> Artificial
<220>
   <223> primer D-1
<400> 5
   catgggttgc ttcgcgcgca agacg 25
<210> 6
   <211> 23
   <212> DNA
   <213> Artificial
<220>
   <223> primer D-2
<400> 6
   cgaagccggc aaaggcggca gtc 23
<210> 7
   <211> 22
   <212> DNA
   <213> Artificial
<220>
   <223> primer D-3
<400> 7
   ccactaaact atgttctcag gc 22
<210> 8
   <211> 28
   <212> DNA
   <213> Artificial
<220>
   <223> primer 12-f
<400> 8
   tctagaatgg cacctcccaa cactattg 28
<210> 9
   <211> 30
   <212> DNA
   <213> Artificial
<220>
   <223> primer 12-r
<400> 9
   aagcttttac ttcttgaaaa agaccacgtc 30
<210> 10
   <211> 29
   <212> DNA
   <213> Artificial
<220>
   <223> primer 6-f
<400> 10
   tctagaatgg ctgctgctcc cagtgtgag 29
<210> 11
   <211> 29
   <212> DNA
   <213> Artificial
<220>
   <223> primer 6-r
<400> 11
   aagcttttac tgtgccttgc ccatcttgg 29
<210> 12
   <211> 28
   <212> DNA
   <213> Artificial
<220>
   <223> primer GLELO-f
<400> 12
   tctagaatgg agtcgattgc gcaattcc 28
<210> 13
   <211> 30
   <212> DNA
   <213> Artificial
<220>
   <223> primer GLELO-r
<400> 13
   gagctcttac tgcaacttcc ttgccttctc 30
<210> 14
   <211> 30
   <212> DNA
   <213> Artificial
<220>
   <223> primer 5-f
<400> 14
   tctagaatgg gtgcggacac aggaaaaacc 30
<210> 15
   <211> 30
   <212> DNA
   <213> Artificial
<220>
   <223> primer 5-r
<400> 15
   aagcttttac tcttccttgg gacgaagacc 30
<210> 16
   <211> 1203
   <212> DNA
   <213> Mortierella alpina
<400> 16
   atggcacctc ccaacactat tgatgccggt ttgacccagc gccatatcag cacctcggcc     60
   gccccaacct ctgccaagcc cgccttcgag cgcaactacc agctccctga gttcaccatc    120
   aaggagatcc gtgagtgcat ccctgcacac tgctttgagc gctccggtct ccgtggtctt    180
   tgccacgttg ctattgatct gacctgggcc tcgctcttgt tcctggctgc gacccagatc    240
   gacaagttcg agaacccttt gatccgctac ttggcctggc ctgcgtattg gatcatgcag    300
   ggtattgttt gcaccggtat ctgggtattg gcacacgaat gtggtcatca gtccttctcg    360
   acctccaaga cccttaacaa cactgtcggc tggatcttgc actcgatgct cttggtccct    420
   taccactcct ggagaatctc gcactcgaag caccacaagg ccactggcca catgaccaag    480
   gaccaggtct ttgttcccaa gacccgctct caggttggct tgccccccaa ggagaatgtt    540
   gcagttgccg ttcaggagga ggatatgtcc gtgcacctgg atgaggaggc ccccattgtg    600
   actttgttct ggatggtgat tcagttcctg ttcggatggc ctgcgtacct tattatgaac    660
   gcctctggtc aagactatgg ccgctggacc tcgcacttcc acacctactc tcctatcttt    720
   gagccccgca actttttcga cattatcatt tcggatctcg gtgtgttggc tgctcttggt    780
   accttgatct acgcctccat gcagctctcg ctcttgaccg tgaccaagta ctacattgtc    840
   ccctacttgt ttgtcaactt ctggttggtc ctgatcacct tcttgcagca caccgaccct    900
   aagctgcccc attaccgtga gggtgcctgg aacttccagc gtggagccct ctgcaccgtt    960
   gaccgctcgt tcggcaagtt cttggaccat atgttccacg gcattgtcca tacccatgta   1020
   gcccatcact tgttctcgca gatgccgttc taccatgctg aggaagccac ccatcatctc   1080
   aagaaactgc tgggagagta ctacgtctat gacccatcgc cgattgttgt tgcggtctgg   1140
   aggtcgttcc gtgaatgccg attcgtggaa gaccatggag acgtggtctt tttcaagaag   1200
   taa                                                                  1203
<210> 17
   <211> 1374
   <212> DNA
   <213> Mortierella alpina
<400> 17
   atggctgctg ctcccagtgt gaggacgttt actcgggccg agattttgaa tgccgaggcc      60
   ctgaatgagg gcaagaagga tgccgaggca ccctttctga tgatcattga caacaaggtg     120
   tacgatgtcc gcgagtttgt ccctgatcat cccggtggaa gtgtgattct cacgcacgtt     180
   ggcaaggacg gcactgacgt ctttgacact ttccaccccg aggctgcttg ggagactctt     240
   gccaactttt acgttggtga tattgatgag agcgatcgtg ccatcaagaa tgatgacttt     300
   gcggccgagg ttcgcaagct gcgcaccttg ttccagtccc ttggctacta cgactcgtcc     360
   aaggcatact atgccttcaa ggtctcgttc aacctctgca tctggggctt gtcgactttc     420
   attgttgcca agtggggcca gacctcgacc ctcgccaacg tgctctcggc tgcgctcttg     480
   ggtctcttct ggcagcagtg cggatggttg gcgcacgact ttttgcacca ccaggtcttc      540
   caggaccgtt tctggggtga tcttttcggc gccttcttgg gaggtgtctg ccagggtttc     600
   tcgtcctcct ggtggaagga caagcacaac actcaccacg ctgctcccaa cgtccacggc     660
   gaggatcccg acattgacac tcaccctctg ttgacctgga gtgagcatgc tctggagatg     720
   ttctcggatg ttcctgacga ggagctgacc cgtatgtggt cgcgcttcat ggtcctcaac     780
   cagacctggt tctacttccc cattctctcg tttgcccgtc tgtcctggtg cctccagtcc     840
   attatgcttg ttctgcccaa cggtcaggcc cacaagccct ctggagcgcg tgtgcccatt     900
   tcgttggtcg agcagctgtc tctggctatg cactggacct ggtacctcgc caccatgttc     960
   ctgttcatta aggatcccgt caacatgatt gtgtactttt tggtgtcgca ggctgtttgc    1020
   ggcaacttgt tggcgattgt gttctcgctc aaccacaacg gcatgcctgt gatctccaag    1080
   gaggaagcgg tcgatatgga cttcttcacc aagcagatca tcacgggtcg tgatgttcac    1140
   cctggtctgt ttgccaactg gttcacgggt ggattgaact accagattga gcaccacttg    1200
   ttcccttcga tgccccgcca caacttttca aagatccagc ctgctgtcga gactttgtgc    1260
   aaaaagtacg gtgtccgata ccataccact ggtatgatcg agggaactgc agaggtcttt    1320
   agccgtttga acgaggtctc caaggcggcc tccaagatgg gcaaggcaca gtaa          1374
<210> 18
   <211> 957
   <212> DNA
   <213> Mortierella alpina
<400> 18
   atggagtcga ttgcgcaatt cctcccctca aagatgccgc aagatctgtt tattgacctt 60
   gcaagggcca tcggtgtcca ggccgcaccc tatgtcgacc ctctcgaggc agcgcttgtg 120
   gcccaggccg agaagttctt ccccacggtc gttcatcaca cgcgcggctt tttggtcgcg 180
   gtcgagtcac ccttggcccg tgagctgccc ttgatgaacc ccttccacgt gctgttgatc 240
   gcgctcgctt acttggtcac ggtctttgtg ggcatgcaga tcatgaagaa ctttgaacgg 300
   ttcgaggtca agacgttctc gctcttccac aacttttgtc tggtctcgat cagtgcctac 360
   atgtgcggcg ggatcttgta cgaggcttac caggccaact atggactgtt tgagaacgcg 420
   gccgatcata ccgtccaggg tcttcctatg gccaagatga tctggctctt ctacttctcc 480
   aagatcatgg agtttgtcga caccatgatc atggtcctta agaagaacaa ccgccagatc 540
   tcgttcttgc acgtctacca ccacagctcc atcttcacca tctggtggtt ggtcaccttt 600
   gttgcaccca atggtgaagc ctacttctcg gctgcgttga actcgttcat ccacgtgatc 660
   atgtacggct actacttcct gtccgccttg ggcttcaagc aggtgtcgtt catcaagttc 720
   tacatcacgc gttcgcagat gacgcagttc tgcatgatgt cgatccagtc ctcctgggac 780
   atgtatgcca tgaaggtgct tggccgcccc ggatacccct tcttcatcac cgccctgctt 840
   tggttctaca tgtggaccat gctcggactc ttctacaact tctacagaaa gaacgccaag 900
   ttggccaagc aggccaagat cgatgctgcc aaggagaagg caaggaagtt gcagtaa 957
<210> 19
   <211> 1341
   <212> DNA
   <213> Mortierella alpina
<400> 19
   atgggtgcgg acacaggaaa aaccttcacc tggcaagaac tcgcggcgca taacaccgag 60
   gacagcctcc ttttggctat ccgtggcaat gtatacgatg tcacaaagtt cttgagccgt 120
   catcctggtg gaacggatac tctcttgctc ggagctggcc gagatgtcac tccggttttt 180
   gagatgtacc acgagtttgg agctgcagag gctatcatga agaagtacta tgttggcaca 240
   ctggtctcaa atgagttgcc catcttccca gagccaacgg tgttccataa gaccatcaag 300
   ggcagagttg aggcatactt taaggaccgg aacatggatt ccaagaacag accagagatc 360
   tggggacgat atgctctcat ctttggatcc ttgatcgcct cttactacgc gcagctcttt 420
   gtaccgttcg tggtcgaacg tacatggctc caggtggtgt ttgctatcat catgggattt 480
   gcgtgcgcgc aagtcggatt gaaccctctt cacgatgcct cccacttttc agtgacccac 540
   aaccccaccg tttggaagat tctcggagcc acgcacgact ttttcaacgg agcatcgtat 600
   ctcgtgtgga tgtaccaaca tatgctcggc catcatccct ataccaacat tgctggagct 660
   gatcccgatg tgtcgacctc tgagcccgat gttcgtcgta tcaagcccaa ccaaaagtgg 720
   ttcgtcaacc acatcaacca gcacatgttt gttcctttcc tgtacggact gctggcgttc 780
   aaggtgcgca tccaggacat caacatcttg tactttgtca agaccaatga cgccattcgt 840
   gtcaacccca tctcgacttg gcacaccgtc atgttctggg gcggaaaggc cttctttgtc 900
   tggtaccgct tgatcgttcc tatgcagtat ctgcccctga gcaaggtgtt gctcttgttt 960
   accgtcgcag acatggtctc ttcttactgg ctggcgctga ccttccaggc gaaccacgtt 1020
   gttgaggagg ttcagtggcc attgcctgat gagaatggaa tcatccaaaa ggattgggca 1080
   gccatgcagg tcgagactac tcaggattac gcccacgatt cgcacctctg gaccagcatc 1140
   acgggcagct tgaactacca agccgttcat catctgttcc cgaacgtttc ccagcatcac 1200
   taccctgata tcctggctat catcaaggac acctgcagcg agtacaaggt gccatacctc 1260
   gtcaaggata ccttttggca agcgtttgct tcacatttgg agcacttgcg tgtgcttggt 1320
   cttcgtccca aggaagagta a 1341
<210> 20
   <211> 20
   <212> DNA
   <213> Artificial
<220>
   <223> primer MaGAPDHpfw
<400> 20
   cacaccacac attcaacatc 20
<210> 21
   <211> 24
   <212> DNA
   <213> Artificial
<220>
   <223> primer D2
<400> 21
   cgaagccggc aaaggcggca gtcg 24
<210> 22
   <211> 25
   <212> DNA
   <213> Artificial
<220>
   <223> primer D1
<400> 22
   catgggttgc ttcgcgcgca agacg 25
cgaagccggc aaaggcggca gtcg 24
<210> 23
   <211> 20
   <212> DNA
   <213> Artificial
<220>
   <223> primer PD4P
<400> 23
   cgcatcccgc aaacacacac 20